# EUROPÄISCHE PATENTSCHRIFT

(11) **EP 0 355 041 B1**
(45) Veröffentlichungstag und Bekanntmachung des Hinweises auf die Patenterteilung: **20.09.1995**
(21) Anmeldenummer: 89250013.3
(22) Anmeldetag: 11.08.1989
(51) Int. Cl.: C07D 487/22, A61K 49/00

(54) **13,17-Propionsäure- und Propionsäurederivat-substituierte Porphyrin-Komplexverbindungen, Verfahren zu ihrer Herstellung und diese enthaltende pharmazeutische Mittel**
Complex porphyrine compounds substituted in position 13,17 by propionic acid and propionic-acid derivatives, process for their preparation and pharmaceutical compositions containing same
Composés complexes de porphyrine substituée en 13,17 par l'acide propionique et les dérivés d'acide propionique, procédé pour leur préparation et compositions pharmaceutiques les contenant

(30) Priorität: 13.08.1988 DE 3827940
(43) Veröffentlichungstag der Anmeldung: 21.02.1990
(73) Patentinhaber: SCHERING AKTIENGESELLSCHAFT, 13342 Berlin (DE)
(72) Erfinder: Niedballa, Ulrich, Dr., D-1000 Berlin 33 (DE); Gries, Heinz, Dr., D-1000 Berlin 31 (DE); Conrad, Jürgen, Dr., D-1000 Berlin 41 (DE); Speck, Ulrich, Prof., D-1000 Berlin 28 (DE)

(56) Entgegenhaltungen:
- EP-A- 0 175 617
- EP-A- 0 213 272
- LIEBIGS ANNALEN DER CHEMIE, Nr. 2, 1984, Seiten 204-210, Weinheim, DE; J.-H.FUHRHOP et al.: "Hydrogen evolution from viologen radicals and fromphotochemically reduced tin(IV) porphyrins in the presence of colloidalplatinum"
- CANADIAN JOURNAL OF CHEMISTRY, Band 57, Nr. 22, 1979, Seiten 2916-2922, Ottawa,CA; G. PAQUETTE et al.: "Cinétique de la formation de la métalloporphyrineCu(II)-dérivé tétra éthylènediamino de la protoporphyrine IX (ENP) en milieuaqueux"
- BIOINORGANIC CHEMISTRY, Band 4, Nr. 1, 1971, Seiten 21-35, New York, US; W.I.WHITE et al.: "A homologous series of water-soluble porphyrins andmetalloporphyrins: Synthesis, dimerization, protonation and self-complexation"
- JOURNAL OF THE AMERICAN CHEMICAL SOCIETY, Band 108, Nr. 14, 1986, Seiten 3740-3744, Washington, DC, US; G.B. KOLSKI et al.: "Temperature-jump investigationof the kinetics of imidazole substitution on an iron(III) porphyrin in aqueoussolution"

## Beschreibung

Die Erfindung betrifft den in den Patentansprüchen gekennzeichneten Gegenstand, das heißt neue Porphyrin-Komplexverbindungen mit Propionsäure- oder Propionsäurederivat-Substituenten in den 13- und 17-Positionen des Porphyringerüstes, diese Verbindungen enthaltende pharmazeutische Mittel, ihre Verwendung in Diagnostik und Therapie sowie Verfahren zur Herstellung dieser Verbindungen und Mittel.

Die Anwendung von Komplexbildnern oder Komplexen bzw. deren Salzen in der Medizin ist seit langem bekannt. Als Beispiele seien genannt:

Komplexbildner als Stabilisatoren pharmazeutischer Präparate, Komplexe und deren Salze als Hilfsmittel zur Verabreichung schlecht löslicher Ionen (z.B. Eisen), Komplexbildner und Komplexe (bevorzugt Calcium- oder Zink-), gegebenenfalls als Salze mit anorganischen und/oder organischen Basen, als Antidots zur Entgiftung bei versehentlicher Inkorporation von Schwermetallen oder deren radioaktiven Isotopen und Komplexbildner als Hilfsmittel in der Nuklearmedizin unter Verwendung radioaktiver Isotope wie ^{99m}Tc für die Szintigraphie sind bekannt.

Die Patentschrift EP 175 617 beschreibt Konjugate aus Antikörpern und Therapeutika, unter denen auch Porphyrine genannt sind, zur Behandlung von zellulären Störungen.

In "Liebigs Annalen der Chemie" Nr. 2 (1984), Seiten 204-210; "Canadian Journal of Chemistry" Band 57, Nr. 22 (1979), Seiten 2916-2922; "Bioinorganic Chemistry" Band 4, Nr. 1 (1971), Seiten 21-35; "Journal of the American Chemical Society" Band 94, Nr. 11 (1972), werden Porphyrin-Verbindungen für in-vitro Untersuchungen (reaktionsmechanistische und kinetische Messungen) verwendet.

In den Patentschriften EP71564, EP 130934 und DE-OS 3401052 sind neuerdings Komplexe und Komplexsalze als Diagnostika, vorwiegend als NMR-Diagnostika, vorgestellt worden.
Diese Komplexe und Komplexsalze sind recht gut verträglich und gewährleisten eine weitestgehende vollständige Ausscheidung der Ionen. Sie erfüllen jedoch noch nicht optimal alle die relative Wirksamkeit eines NMR-Kontrastmittels bestimmenden Kriterien, von denen vor allem zu nennen sind:
eine starke NMR-Aktivität (Relaxivität), so daß das Kontrastmittel in möglichst geringen Konzentrationen die Relaxationszeiten der Protonen im Gewebewasser und anderer für die NMR relevanter Kerne (wie Phosphor, Fluor, Natrium) in vivo erniedrigt und damit die Lokalisation von Tumoren durch Erhöhung der Signalintensität des mit Hilfe des Kernspintomographen erhaltenen Bildes ermöglicht;
eine möglichst selektive Anreicherung und/oder Retention des Kontrastmittels am Zielorgan bzw. cancerösem Gewebe; ausreichende Wasserlöslichkeit; hohe Wirksamkeit; gute Vertraglichkeit; gute chemische und biochemische Stabilität.

Für die Bildgebung relevant sind dabei vor allem die beiden erstgenannten Punkte. Da die Relaxationszeiten zwischen den Geweben sich meistens nur um den Faktor 2-3 unterscheiden (T.E. Budinger und P.C, Lauterbur, Science 226, pp 288-298, 1984; J.M.S. Hutchinson und F.W. Smith in Nuclear Magnetic Resonance Imaging Edit. C.L. Partain et al., pp 231-249, Saunders, New York 1983) und die Komplexe und Komplexsalze der genannten Patentschriften im allgemeinen mit dem Nachteil behaftet sind, daß sie sich nur relativ unspezifisch im Extrazellulärraum verteilen und daher nicht immer für eine Erkennung pathologisch veränderter Gewebe taugen, besteht ein Bedarf nach vor allem selektiv-bindenden, tumorspezifischen Verbindungen, die sich in der Diagnostik verwenden lassen.

Es ist nun seit einigen Jahren bekannt, daß sich Porphyrinderivate selektiv in menschlichen und tierischen Tumoren anreichern (D. Kessel und T.-H. Chou, Cancer Res. 43, pp 1994-1999, 1983, P. Hambright, Bioinorg. Chem. 5, pp 87-92, 1975; R. Lipson et al., Cancer 20, pp. 2250-2257, 1967; D. Sanderson et al.,, Cancer 30, pp. 1368-1372, 1972). Erste Ansätze, diese Verbindungsklasse auch als Diagnostika einzusetzen sind ebenfalls beschrieben worden (J. Winkelmann et al., Cancer Research 27, pp. 2060-2064, 1967; N. J. Patronas et al., Cancer Treatment Reports 70, pp. 391-395, 1986). Bei diesen Verbindungen hat sich als paramagnetisches Metall nur das Mangan(III)-Ion als geeignet erwiesen.

Die bisher beschriebenen Verbindungen sind jedoch weit davon entfernt die oben genannten Kriterien zufriedenstellend zu erfüllen; besondere Aufmerksamkeit verlangt immer noch ihre mangelnde Anreicherung in den Zielorganen. Eine Verbesserung dieser Eigenschaft sollte gleichzeitig die bestehenden Probleme mit der Toxizität und Verträglichkeit der vorbekannten Verbindungen reduzieren helfen.

Für das Tumor Imaging mit Radioisotopen sind neuerdings Derivate des Deuteroporphyrins vorgeschlagen worden, die als zusätzliche komplexbildende Gruppen Polyaminopolycarbonsäuren über Ethylenglycolbrücken an das Porphyringerüst gebunden enthalten (Photochemistry and Photobiology Vol. 46, pp 783-788 (1987)).
Solche Porphyrinester sind jedoch für eine parenterale Anwendung am Patienten -insbesondere für die NMR-Diagnostik - wenig geeignet, da die daraus erhältlichen Injektionslösungen wegen hydrolytischer Spaltungen weder hitzesterilisierbar noch über einen ausreichenden Zeitraum lagerfähig sind.

Es besteht daher weiterhin für vielfältige Zwecke ein Bedarf an stabilen, gut löslichen, aber auch besser verträglichen, selektiver bindenden, gut zugänglichen, über eine größere chemische Variationsbreite der Substituenten (die z.B. den Einbau anderer Metalle als Mangan bzw. mehrerer, auch unterschiedlicher, Metalle ermöglicht und damit gleichzeitig auch zu einer Steuerung der Eigenschaften und Anwendungen der Verbindungen führt) verfügenden Komplexverbindungen, die für die Diagnostik und/oder auch Therapie von Tumoren geeignet sind.

Der Erfindung liegt somit die Aufgabe zugrunde, diese Verbindungen und pharmazeutischen Mittel zur Verfügung zu stellen, sowie ein Verfahren zu ihrer Herstellung zu schaffen.
Diese Aufgabe wird durch die Erfindung gelöst.
Es wurde gefunden, daß sich Porphyrin-Komplexverbindungen, bestehend aus mindestens einem Porphyrinliganden und mindestens einem Ion eines Elements der Ordnungszahlen 5, 13, 21-32, 37-39, 42-44, 49, 50 oder 57-83 sowie gegebenenfalls Kationen anorganischer und/oder organischer Basen, Aminosäuren oder Aminosäureamide, überraschenderweise hervorragend zur Herstellung von NMR-, Röntgen- und Radio-Diagnostika eignen.

Die erfindungsgemäßen Komplexverbindungen weisen als Porphyrinliganden Verbindungen der allgemeinen Formel I auf 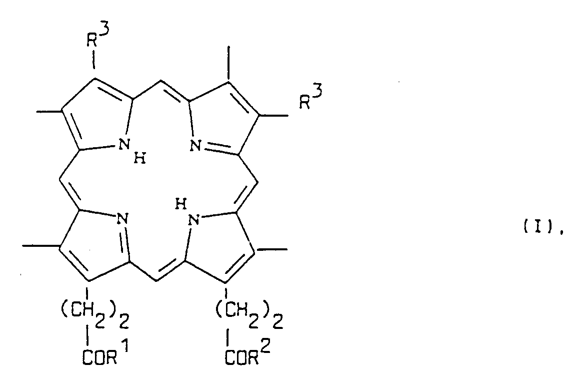
worin
- R¹ und R²: unabhängig voneinander für die OH- oder (NH)ₓ-[Q-(NH)_{y}]_{w}-W-Gruppe, wobei
- X und y: die Ziffern 0, 1 oder 2,
- w: die Ziffern 0 oder 1,
- Q: eine C₀-C₂₀-Alkylengruppe,
- W: ein Wasserstoffatom oder die Gruppierung V-K bedeuten, wobei
- V: eine gegebenenfalls Imino-, Polyethylenoxy-, Phenylenoxy-, Phenylenimino-, Amido-, Hydrazido-, Estergruppe(n), Sauerstoff-, Schwefel-und/oder Stickstoff-Atom(e) enthaltende gegebenenfalls durch Hydroxy-, Mercapto-, Imino-, Epoxy-, Oxo-, Thioxo- und/oder NHK-gruppe(n) substituierte geradkettige, verzweigte, gesättigte oder ungesättigte C₀-C₂₀-Alkylengruppe,
- K: ein Wasserstoffatom oder ein Komplexbildner der allgemeinen Formeln IA, IB, IC oder ID 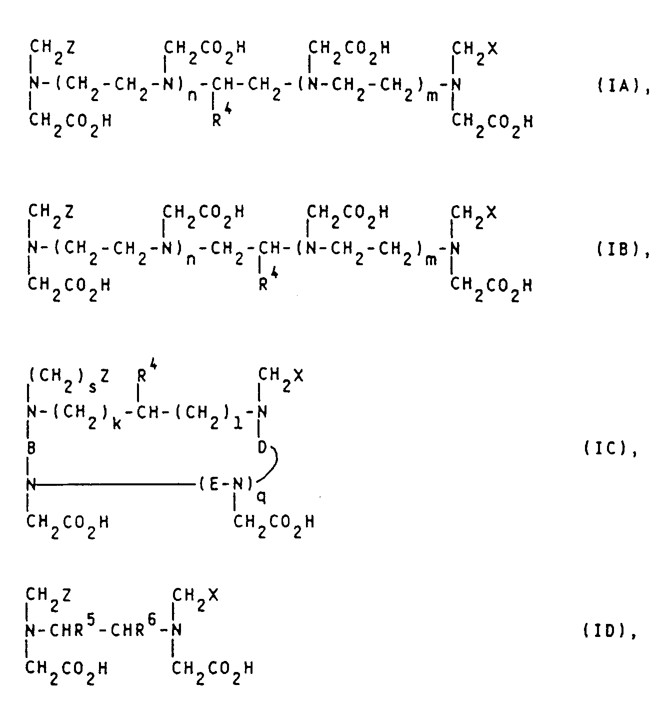 bedeuten,
mit der Maßgabe, daß entweder Z oder R⁴ an V gebunden ist, wobei
n und m jeweils für die Ziffern 0, 1, 2, 3 oder 4, wobei n und m zusammen nicht mehr als 4 ergeben,
k für die Ziffern 1, 2, 3, 4 oder 5,
l für die Ziffern 0, 1, 2, 3, 4 oder 5,
q für die Ziffern 0, 1 oder 2,
s für die Ziffern 0 oder 1,

- B, D und E,: die gleich oder verschieden sind, jeweils für die Gruppe 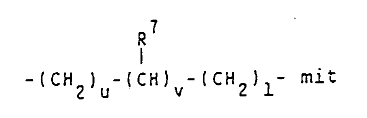
- R⁷: in der Bedeutung von Wasserstoff oder einer gegebenenfalls Sauerstoff- und/oder Stickstoffatom(e) enthaltenden gegebenenfalls durch Hydroxy-und/oder Aminogruppe(n) substituierten geradkettigen, verzweigten, gesättigten oder ungesättigten C₁-C₂₀-Alkylgruppe, u in der Bedeutung der Ziffern 0, 1, 2, 3, 4 oder 5, v in der Bedeutung der Ziffern 0 oder 1, wobei B, D und E jeweils mindestens 2 und maximal 5 Kohlenstoffatome enthalten,
- Z: für den Rest -CO₂H oder die Gruppe 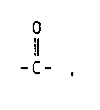
- X: für den Rest -CO₂H oder 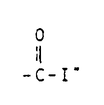 mit I'' in der Bedeutung eines weiteren Porphyrinliganden der allgemeinen Formel I, wobei der in R¹ und R² enthaltene Rest W hier für eine direkte Bindung steht,
- R⁴: für eine direkte Bindung oder ein Wasserstoffatom,
- R⁵ und R⁶: gemeinsam für eine gegebenenfalls durch 1-2 Hydroxy- oder 1-3 C₁-C₄-Alkylgruppen substituierte Dimethylen- oder Trimethylen-methingruppe [falls Z für den Rest -CO₂H steht) bzw. gemeinsam für eine gegebenenfalls durch 1-2 Hydroxy- oder 1-3 C₁-C₄-Alkylgruppen substituierte Trimethylen- oder Tetraamethylengruppe (falls Z für die Gruppe 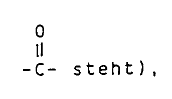 stehen,
mit der Maßgabe, daß nicht gleichzeitig x und w für die Ziffer 0 und V für eine C₀-Alkylenkette stehen und daß Z nur dann für die Gruppe 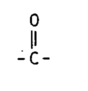 steht, wenn R⁴ gleichzeitig ein Wasserstoffatom bedeutet, und daß Z nur dann für den Rest -CO₂H steht, wenn R⁴ gleichzeitig eine direkte Bindung und s die Ziffer 1 bedeuten,
- R³: für den Rest -CH(R¹)CH₃ oder -CH₂CH₂R¹
stehen, mit der Maßgabe, daß R¹ und R² nicht gleichzeitig für die Hydroxygruppe stehen, daß falls R¹ und R² jeweils für die Gruppe -NH(CH₂)ᵣNH₂ mit r in der Bedeutung der Ziffern 2, 3, 4 oder 6 stehen der Substituent R¹ im Rest R³ nicht ebenfalls für diese Gruppe steht, daß der Substituent R¹ im, Rest R³ nur für Wasserstoff steht, wenn W in R¹ und R² die Gruppierung V-K bedeutet und daß gewünschtenfalls ein Teil der CO₂H-Gruppen als Ester und/oder Amid sowie die in 3- und/oder 8-Stellung vorhandene Hydroxygruppe gewünschtenfalls als Ether oder Ester vorliegt.

Unter einer C₀-Alkylengruppe ist eine direkte Bindung zu verstehen.

Die erfindungsgemäßen Komplexverbindungen umfassen insgesamt drei Gruppen von Verbindungen:
a) Verbindungen, die ein Metallion im Porphyrin-Liganden enthalten;
b) Verbindungen, die mindestens ein Metallion im Komplexbildner-Rest K enthalten; und
c) Verbindungen, die sowohl Metallionen im Porphyrinliganden als auch im Komplexbildner-Rest K gebunden enthalten, wobei die Metallionen unterschiedlich sein können.

Sind die erfindungsgemäßen Mittel zur Anwendung in der NMR-Diagnostik bestimmt, so müssen paramagnetische Metallionen im Komplex vorhanden sein. Dies sind insbesondere die zwei- und dreiwertigen Ionen der Elemente der Ordnungszahlen 21-29, 42, 44 und 57-70. Geeignete Ionen sind beispielsweise das Chrom(III)-, Mangan(II)-, Mangan(III)-, Eisen(II)-, Cobalt(II)-, Nickel(II)-, Kupfer(II)-, Praseodym(III), Neodym(III)-, Samarium(III)- und Ytterbium(III)-ion. Wegen ihres hohen magnetischen Moments sind besonders bevorzugt das Gadolinium(III), Terbium(III)-, Dysprosium(III)-, Holmium(III)-, Erbium(III)- und Eisen(III)ion.

Für die Verwendung der erfindungsgemäßen Mittel in der Nuklearmedizin müssen die Metallionen radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium, Thallium und Iridium.

Es ist auch möglich, daß das radioaktive Isotop vom Porphyrinliganden komplexiert wird [Fall a) siehe oben], vom Komplexbildner-Rest K [Fall b)] oder daß es vom Komplexbildner-Rest K chelatisiert wird während gleichzeitig der Porphyrinligand ein anderes Metallion, zum Beispiel Mangan(III), enthält [Fall c)].
Sind die erfindungsgemäßen Mittel zur Anwendung in der Röntgen-Diagnostik bestimmt, so muß sich zumindest ein Metallion im Komplex von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Ein wesentlicher Vorteil der erfindungsgemäßen, den Komplexbildner-Rest K enthaltenden Metallkomplexe ist, daß bei diesen der vom Metallion bewirkte diagnostische bzw. therapeutische Effekt durch den Einbau eines weiteren Metallions verstärkt werden kann, bzw. durch den Einbau eines anderen, vom ersten unterschiedlichen Metallions, insbesondere die physikalischen Eigenschaften durch eine Verstärkung der magnetischen Effekte und/oder der Röntgenstrahl-Absorption der Komplexverbindungen verbessert werden können.

Überraschend ist hierbei, daß die wesentlichen, die Wirksamkeit dieser pharmazeutischen Mittel bestimmenden Eigenschaften, wie zum Beispiel vor allem die hohe Selektivität und Anreicherung der Komplexe, erhalten bleiben oder verbessert werden.

Mit Hilfe der erfindungsgemäßen Komplexverbindungen können überraschenderweise nicht nur Tumorgewebe und einzelne Organe, wie zum Beispiel Leber und Nieren, sondern auch Blutgefäße ohne Anwendung spezieller Puls-Sequenzen in-vivo dargestellt werden, womit sie unter anderem als Perfusionsagentien Verwendung finden können.

Als Beispiel für die im Porphyringerüst gebundenen Ionen seien die Metalle Aluminium, Mangan, Eisen, Cobalt, Nickel, Kupfer, Zink, Gallium, Technetium, Indium, Zinn und Thallium genannt. Bevorzugt sind die Metalle Aluminium, Cobalt, Zink, Gallium, Technetium, Indium, Zinn und insbesondere Mangan.

Als Beispiele für die ans Porphyringerüst gebundenen Ionen seien die Übergangsmetalle, die Seltenen Erden und Bor genannt.

Die für V und Q stehende Alkylengruppe bzw. die für R⁷ stehende Alkylgruppe kann geradkettig, verzweigt, cyclisch, aliphatisch, aromatisch oder arylaliphatisch sein, bis zu 20 Kohlenstoffatome aufweisen und - im Falle von V - gegebenenfalls -NH-, -O-, -S-, -H-, -CO-O-, -O-CO-, (O-CH₂CH₂-)poly, -NH-CO-, -CO-NH-, -NH-NH-, -C₆H₄-NH-, -C₆H₄-O-, -C₆H₄- enthalten, Strukturen wie z.B. -CO-CH-(NH₂)-CH₂NH-, -S-(CH₂)₂-NH- aufweisen oder durch Hydroxy-, Mercapto-, Imino-, Epoxy-, Oxo-, Thioxo- und/oder -NHK-Gruppen substituiert sein. Bevorzugt sind die geradkettigen Mono-bis Decamethylengruppen sowie C₁-C₄-Alkylenphenylgruppen.

Zur Verdeutlichung seien folgende Alkylengruppen beispielhaft genannt:
-(CH₂)₂NH-; -CH₂-O-C₆H₄-CH₂-; -CH₂-CH(OH)-CH₂-O-6₆H₄-CH₂-; -C(=NH)-O-C₆H₄-CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂; -CH₂-CO-NH-(CH₂)₃-O-CH₂-; -CH₂-CO-NH-NH-; -CH₂-CO-NH-(CH₂)₂-; -CH₂-CO-NH(CH₂)₁₀-; -CH₂-CO-NH-(CH₂)₂-S-; -(CH₂)₄-NH-CO-(CH₂)₈-; -CH₂-CO-NH-(CH₂)₃-NH; -(CH₂)₃-NH-; -(CH₂)NH-C(=S)-NH-C₆H₄-CH₂-; -CO-CH(NHK)-CH₂NHK-; -CH(CH₃)-S-(CH₂)₂-NH-; -(CH₂)₂NH-CO-CH₂-(OCH₂CH₂)₄₃OCH₂.

Als Beispiele für die Komplexbildner-Reste K seien diejenigen der Ethylendiamintetraessigsäure, Diethylentriaminpentaessigsäure, trans-1,2-Cyclohexandiamintetraessigsäure, 1,4,7,10-Tetraazacyclododecantetraessigsäure, 1,4,7-Triazacyclononan-triessigsäure, 1,4,8,11-Tetraazatetradecantetraessigsäure und 1,5,9-Triazacyclododecantriessigsäure genannt, die über (jeweils in K enthaltend) ein Kohlenstoffatom oder eine Carbonylgruppe an die jeweiligen Porphyrinderivate gebunden sind. Gewünschtenfalls kann ein Teil der Carbonsäuren als Ester und/oder Amid vorliegen.

Die in R³ enthaltenen Hydroxygruppen können gewünschtenfalls in veretherter oder veresterter Form vorliegen, wobei als Alkylsubstituent Kohlenwasserstoffe mit 1-6, vorzugsweise 1-4 C-Atomen, wie zum Beispiel Methyl-, Ethyl-, Propyl-, Isopropyl-, n-, sec- oder tert.-Butyl-, Isobutyl-, Pentyl-, Hexyl-, Cyclohexyl- und Phenylreste in Betracht kommen.

Als Acylsubstituent kommen solche mit 1-10 C-Atomen, z.B. der Formyl-, Acetyl-, Propionyl-, Isopropionyl-, Butyryl-, Isobutyryl-, Valenyl-, Benzoyl-, Trifluoracetyl-, Mono-, Di- und Trichloracetyl- und Nicotinylrest in frage, wobei der Acetyl- und Benzoylrest bevorzugt sind.

Die restlichen aciden Wasserstoffatome, das heißt diejenigen, die nicht durch das/die Metallion(en) substituiert worden sind, können gegebenenfalls durch Kationen anorganischer und/oder organischer Basen, Aminosäuren oder Aminosäureamide ersetzt bzw. die entsprechenden Säuregruppen ganz oder teilweise in Ester (vorzugsweise solche mit 1 bis 7 C-Atomen) oder Amide (deren Stickstoffatom durch einen oder zwei Kohlenstoffrest(e) - die gerad- oder verzweigtkettig, cyclisch, gesättigt oder ungesättigt, gegebenenfalls durch eine oder mehrere Hydroxy- oder C₁-C₄-Alkoxygruppen substituiert sein könnenb - mit bis zu 10 C-Atomen substituiert sein kann) überführt sein. Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,NDimethylglucamin und insbesondere N-Methyl-glucamin. Geeignete Kationen von Aminosäuren sind beispielsweise die des Lysins, des Arginins und des Ornithins sowie die Amide ansonsten saurer oder neutraler Aminosäuren.

Die Herstellung der erfindungsgemäßen Komplexverbindungen erfolgt dadurch, daß man in Porphyrinen der allgemeinen Formel I' 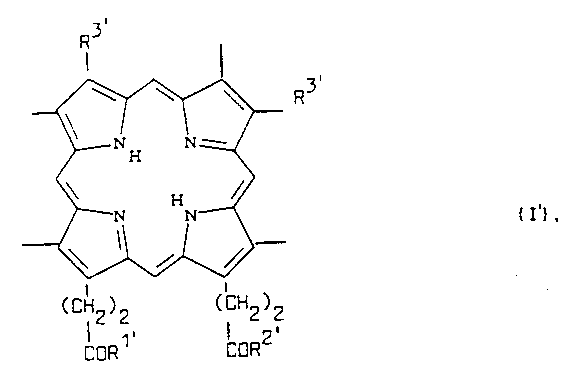
worin
- R^{1'} und R^{2'}: jeweils für die OH- oder O-C₁-C₆-Alkyl-Gruppe und
- R^{3'}: für den Rest -CH(U)CH₃ oder -CH₂CH₂-U mit U in der Bedeutung einer OH- oder O-C₁-C₆-Alkyl-Gruppe oder eines Nucleofugs Nf
stehen
in an sich bekannter Weise, gegebenenfalls wiederholt, mit Verbindungen der allgemeinen Formel II

Y-(NH)ₓ-[Q-(NH)_{y}]_{w}-V¹-H (II),

worin
Y für ein Wasserstoffatom, für Nf oder eine Schutzgruppe und
V¹ für V (falls V² für eine C₀-Alkylenkette steht, siehe unten) oder zusammen mit V² (siehe unten) für V steht,
umsetzt und anschließend, gegebenenfalls nach Abspaltung der Schutzgruppen, gewünschtenfalls
a) die pyrrolischen NH's durch das gewünschte Metallatom substituiert, gegebenenfalls noch vorhandene Schutzgruppen entfernt und anschließend gewünschtenfalls den Rest V²-K, worin V² für V oder für eine aktivierte Form von V (falls V¹ für eine C₀-Alkylenkette steht) oder zusammen mit V¹ für V steht, einführt und danach gewünschtenfalls mit mindestens einem Metalloxid oder Metallsalz eines Elements der Ordnungszahlen 21-32, 37-39, 42-44, 49, 50 oder 57-83 umsetzt oder
b) den Substituenten V²-K, worin V² für V oder für eine aktivierte Form von V (falls V¹ für eine C₀-Alkylenkette steht) oder zusammen mit V¹ für V steht, einführt, gewünschtenfalls mit mindestens einem Metalloxid oder Metallsalz eines Elements der Ordnungszahlen 21-32, 37-39, 42-44, 49, 50 oder 57-83 umsetzt und anschließend gewünschtenfalls die pyrrolischen NH's durch ein Metallatom substituiert oder
c) den Substituenten V²-K worin V² für V oder für eine aktivierte Form von V (falls V¹ für eine C₀-Alkylenkette steht) oder zusammen mit V¹ für V steht, einführt, die pyrrolischen NH's gewünschtenfalls durch ein Metallatom substituiert und anschließend gewünschtenfalls mit mindestens einem Metalloxid oder Metallsalz eines Elements der Ordnungszahlen 21-32, 37-39, 42-44, 49, 50 oder 57-83 umsetzt, wobei zur Herstellung von zwei Porphyrin-Ringe enthaltenden Komplexverbindungen 2 Mole des nach Umsetzung von I' mit II erhaltenen Porphyrins mit einem Komplexbildner K, der zwei V²-Reste enthält, umgesetzt werden,
und gegebenenfalls anschließend in den nach a), b) oder c) erhaltenen Komplexverbindungen noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester und/oder Amide und/oder vorhandene Hydroxygruppen in Ether oder Ester überführt.

V¹ und V² ergeben zusammen den Rest V, wobei sowohl V¹ als auch V² für eine C₀-Alkylenkette stehen kann.

Geeignete Schutzgruppen sind z. B. der Carbobenzoxy- und der t-Butoxycarbonylrest. Als Nucleofug können z.B. Cl-, Br-, CH₃C₆H₄SO₃-, CH₃SO₃- oder der N-oxysuccinimid-Rest dienen.

Die Funktionalisierung der C-13- und/oder C-17-Propionsäureseitenketten zu aminoalkylensubstituierten Porphyrin-C-13- und/oder C-17-amidderivaten (die als Edukte für Porphyrinkomplexe, die den Komplexbildner-Rest K enthalten, dienen können) erfolgt durch Umsetzung der gewünschten Propionsäureketten-tragenden Porphyrine (gegebenenfalls in Form aktivierter Porphyrinsäurederivate wie z.B. Ester, Anhydrid oder Säurechlorid) mit gegebenenfalls substituierten Hydrazinen oder mit endständigen (gegebenenfalls weitere, ebenfalls geschützte Aminogruppen enthaltend) Alkylendiaminen, von denen eine Aminogruppe z.B. in Form des Carbobenzoxy- oder t-Butoxycarbonylrestes geschützt ist. Die Entfernung der Schutzgruppen erfolgt anschließend nach literaturbekannten Methoden, zum Beispiel durch Hydrierung oder Behandlung mit Trifluoressigsäure bzw. mit Salzsäure/Eisessig. Man kann die gewünschte Alkylenkette V auch stufenweise in das Porphyringerüst einbauen, so z.B. zunächst durch eine Amidierungsreaktion mit einem partiell geschützten Diamin, Abspaltung der Schutzgruppe und Umsetzung mit einer eine nucleofuge Gruppe enthaltenden weiteren Verbindung der allgemeinen Formel II, z.B. mit einer Di-t-butoxycarbonylgeschützten 2,3-Diamino-Propionsäure, deren Säuregruppe in Form des Hydroxysuccinimidesters aktiviert ist.

Bei der Funktionalisierung der C-13-Propionsäureseitenkette entsteht jeweils auch die entsprechende isomere C-17-Verbindung mit.

Die Herstellung der erfindungsgemäßen, in den Positionen 3 und 8 funktionalisierten Porphyrinverbindungen erfolgt in an sich bekannter Weise zunächst durch Überführung der entsprechenden Hydroxyverbindungen in Verbindungen der allgemeinen Formel I', in denen U für ein Nucleofug wie z.B. Br steht. Anschließende Umsetzung mit Verbindungen der allgemeinen Formel II, z.B. geschützten Mono-, Diaminen oder auch Mercaptoalkylaminen ergibt die für die Einführung des Komplexbildner-Restes K geeigneten Porphyrin-Komplexe.

Zur Einführung des Komplexbildner-Restes K werden die so erhaltenen Verbindungen in an sich bekannter Weise mit isothiocyanatobenzyl-substituierten Komplex-bildnern umgesetzt (O. Gansow et al., Inorg. Chem. 25, 2772, 1986), amidiert, hydraziniert (Houben-Weyl, Methoden der organischen Chemie, Band VIII/3 Georg Thieme Verlag, Stuttgart (1952), 654 und 676), acyliert (J. March, Advanced Organic Chemistry, McGraw-Hill, 2nd ed.,(1977) 377-382) und/oder alkyliert (Houben-Weyl, Methoden der organischen Chemie, Band VI/3 Georg Thieme Verlag, Stuttgart (1965), 187).
Als Substrat zur Einführung der Einheiten V²-K dienen Verbindungen der allgemeinen Formeln I'A, I'B, I'C, I'D, I''AB, I''C und I''D 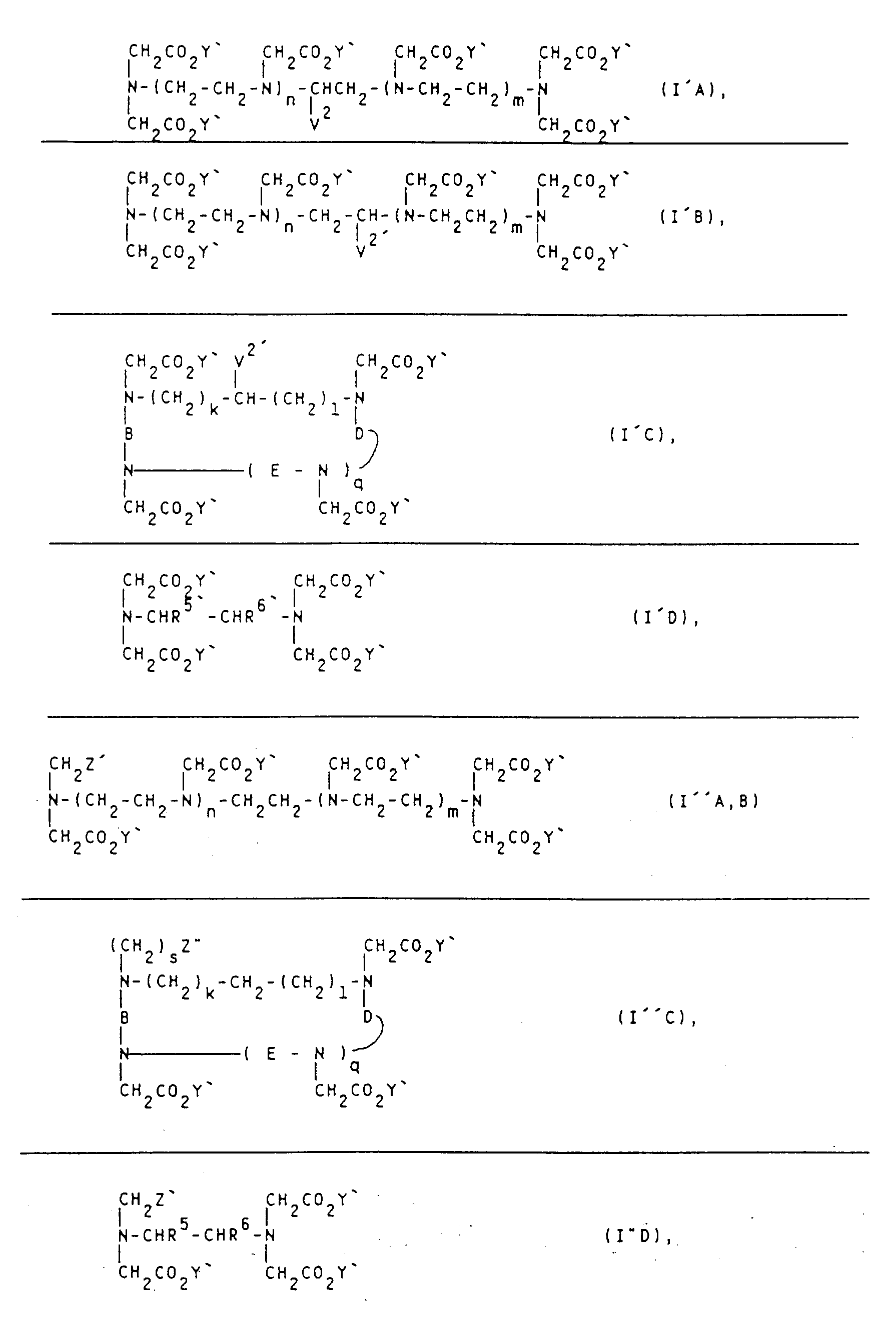
worin V^{2'} für einen in V² umzuwandelnden Substituenten, R^{5'} und R^{6'} für R⁵ und R⁶, die den Substituenten V^{2'} enthalten, Y' für ein Wasserstoffatom oder eine Säureschutzgruppe, Z' für eine aktivierte Carboxylgruppe und Z'' für Z' oder im Falle von s = 0 für Wasserstoff stehen.

Im letztgenannten Fall (I''C mit s=0 und Z''=H) werden die Verbindungen der allgemeinen Formel I''C mit aktivierten Porphyrinsäurederivaten umgesetzt.

Als Beispiel für eine aktivierte Carboxylgruppe seien Anhydrid (dieses kann auch mit der benachbarten Säuregruppe des gleichen Moleküls gebildet sein), p-Nitrophenylsäureester und Säurechlorid genannt.

Als Säureschutzgruppen Y' kommen niedere Alkyl-, Aryl- und Aralkylgruppen, beispielsweise die Methyl-, Ethyl-, Propyl-, Butyl-, Phenyl-, Benzyl-, Diphenylmethyl, Triphenylmethyl-, bis(p-Nitrophenyl)-methylgruppe, sowie Trialkylsilylgruppen in Frage.

Die Abspaltung der Schutzgruppen Y' erfolgt nach den dem Fachmann bekannten Verfahren, beispielsweise durch Hydrolyse, alkalische Verseifung der Ester mit Alkali in wäßrig-alkoholischer Lösung bei Temperaturen von 0°C bis 50°C oder im Fall von tert.-Butylestern mit Hilfe von Trifluoressigsäure.

Die zur Einführung der Komplexbildner-Einheiten vorgenommene Alkylierung bzw. Acylierung wird mit Reagenzien durchgeführt, die den gewünschten K-V- Substituenten (gebunden an eine Fluchtgruppe) enthalten oder aus denen der gewünschte Substituent, gegebenenfalls nach Modifikation durch Folgereaktion(en), durch die Reaktion generiert wird. Als Beispiele für die erstgenannten seien Halogenide, Mesylate, Tosylate und Anhydride genannt. Zur zweiten Gruppe gehören zum Beispiel Oxirane, Thiirane, Azirane, α,β-ungesättigte Carbonylverbindungen oder deren Vinyloge, Aldehyde, Ketone, Isothiocyanate und Isocyanate.

Als Beispiele für Folgereaktionen seien Hydrierungen, Veresterungen, Oxidationen, Veretherungen und Alkylierungen genannt, die nach dem Fachmann bekannten Literaturverfahren durchgeführt werden.

Auf diese Weise werden Porphyrinkomplexe erhalten, die außer einem Metall im Porphyrin-Ringsystem weitere (gleich- oder verschiedenartige) Metalle über die Komplexbildner K in den schwer hydrolysierbaren peripheren Seitenketten stabil binden können.

Sollen Verbindungen mit zwei Porphyrinringen im Molekül synthetisiert werden, so wird entweder ein Porphyrin der allgemeinen Formel I, worin W für ein Wasserstoffatom steht mit einer gegebenenfalls aktivierten Säuregruppe eines K-enthaltenden Porphyrins umgesetzt oder es werden 2 Mole eines den Rest (NH)ₓ-[Q-(NH)_{y}]_{w}-V¹-H enthaltenden Porphyrins der allgemeinen Formel I mit einem bifunktionalisierten Substrat der allgemeinen Formel I''A,B, I''C oder I''D (z.B. Bisanhydrid) umgesetzt.

Die als Edukte benötigten Verbindungen I' sind bekannt (Z.B. Europäische Patentanmeldung Publikations Nr. 0154788) oder können aus den entsprechenden Polyaminen (wobei vorhandene funktionelle Gruppen gegebenenfalls geschützt sind) durch Alkylierung mit einem Ester der allgemeinen Formel III

HalCH₂COOY' (III),

worin Hal für Chlor, Brom oder Jod steht, hergestellt werden.

Die Umsetzung erfolgt in polaren aprotischen Lösungsmitteln wie zum Beispiel Dimethylformamid, Dimethylsulfoxid oder Hexamethylphosphorsäuretriamid in Gegenwart eines Säurefängers, wie zum Beispiel tertiäres Amin (zum Beispiel Triäthylamin, Trimethylamin, N,N-Dimethylaminopyridin, 1,5-Diazabicyclo[4.3.0]-nonen-5(DBN), 1,5-Diazabicyclo[5.4.0]-undecen-5 (DBU), Alkali-, Erdalkalicarbonat oder -hydrogencarbonat (zum Beispiel Natrium-, Magnesium-, Calcium-, Barium-, Kalium- carbonat und -hydrogen-carbonat) bei Temperaturen zwischen -10°C und 120°C, vorzugsweise zwischen 0°C und 50°C.

Die Herstellung der aktivierten Carboxylderivate I''A,B, I''C oder I''D (z.B. gemischtes Anhydrid, N-Hydroxysuccinimidester, Acylimidazole, Trimethylsilylester) erfolgt nach literaturbekannten Methoden [Houben-Weyl, Methoden der organischen Chemie, Georg Thieme Verlag, Stuttgart, Band E 5(1985), 633; Org. React. 12, 157(1962)] oder wird im experimentellen Teil beschrieben.

Die für die Herstellung der Polyamin-polysäuren der allgemeinen Formel I'A als Edukte benötigten entsprechenden Polyamine werden analog literaturbekannten Methoden (zum Beispiel Canad. Patent No. 1 178 951, Eur. I. Med. Chem.-Chim. Ther. 1985,20,509 und 1986, 21,333) hergestellt, indem man von Aminosäuren ausgeht, die in gegebenenfalls ethylenaminsubstituierte Amide (zum Beispiel mit N-(2-Aminoethyl)-carbaminsäurebenzylester) überführt und anschließend (gegebenenfalls nach Abspalten der Schutzgruppen) zu den gewünschten Aminen (vorzugsweise mit Diboran oder Lithiumaluminiumhydrid) reduziert werden.

Will man die Polyamin-Edukte für die Verbindungen der allgemeinen Formel I'B synthetisieren, so ist es notwendig, vor der Reduktion ein derartiges Amid durch Umsetzung mit zum Beispiel Ethyloxamat in einem polaren Lösungsmittel wie zum Beispiel Tetrahydrofuran, Dimethylsulfoxid oder Dimethoxyethan bei einer Temperatur zwischen 50°C und 250°C, vorzugsweise 70°C bis 150°C (gegebenenfalls in einem Druckbehälter) an der α-Aminogruppe zu substituieren, so daß man ein 3-Aza-2-oxo-glutarsäurediamid-Derivat als Zwischenprodukt erhält.

Die Herstellung der als Edukte für I'C bzw. I''C benötigten cyclischen Polyamine erfolgt durch Cyclisierung zweier Reaktanten, von denen (im Falle der Synthese von I'C) der eine V^{2'}-substituiert ist.

Die Cyclisierung wird nach literaturbekannten Methoden, zum Beispiel Org. Synth. 58,86 (1978), Macrocyclic Polyether Syntheses, Springer Verlag, Berlin, Heidelberg, New York 1982. Coord. Chem. Rev. 3,3 (1968), Ann. Chem. 1976, 916, durchgeführt: einer der beiden Reaktanten trägt am Kettenende zwei Fluchtgruppen, der andere zwei Stickstoffatome, die nukleophil diese Fluchtgruppen verdrängen. Als Beispiel seien genannt die Umsetzung von endständigen, gegebenenfalls ein oder zwei Stickstoffatom(e) enthaltenden, Dibrom-, Dimesyloxy-, Ditosyloxy- oder Dialkoxycarbonylalkylenverbindungen mit endständigen, gegebenenfalls ein oder zwei zusätzliche Stickstoffatom(e) in der Alkylenkette enthaltenden Diazaalkylenverbindungen, von denen (im Falle der Synthese von I'C) einer der beiden Reaktanten V^{2'}-substituiert ist.

Die Stickstoffatome sind gegebenenfalls geschützt, zum Beispiel als Tosylate, und werden vor der nachfolgenden Alkylierungsreaktion nach literaturbekannten Verfahren freigesetzt.

Werden Diester in die Cyclisierungsreaktion eingesetzt, so müssen die so erhaltenen Diketoverbindungen nach dem Fachmann bekannten Verfahren, zum Beispiel mit Diboran, reduziert werden.
Als Substituenten V^{2'}, der in V² überführt werden kann, sind unter anderem Hydroxy-und Nitrobenzyl-, Hydroxy-und Carboxyalkyl- sowie Thioalkylreste mit bis zu 20 Kohlenstoffatomen geeignet. Sie werden nach dem Fachmann bekannten Literaturverfahren (Chem. Pharm. Bull. 33,674 (1985), Compendium of Org. Synthesis Vol. 1-5, Wiley and Sons, Inc., Houben-Weyl, Methoden der organischen Chemie, Band VIII, Georg Thieme Verlag, Stuttgart, J. Biochem. 92, 1413,1982) in die gewünschten Substituenten ( zum Beispiel mit der Amino-, Hydrazino-, Hydrazinocarbonyl-, Epoxid-, Anhydrid-, Halogeno-, Halogenocarbonyl-, Mercapto-, Isothiocyanatgruppe als funktioneller Gruppe) umgewandelt, wobei im Falle des Nitrobenzylrestes zunächst eine katalytische Hydrierung (zum Beispiel nach P.N. Rylander, Catalytic Hydrogenation over Platinum Metals, Academic Press 1967) zum Aminobenzylderivat vorgenommen werden muß.
Beispiele für die Umwandlung von an aromatische oder aliphatische Reste gebundenen Hydroxy- oder Aminogruppen sind die in wasserfreien, aprotischen Lösungsmitteln wie Tetrahydrofuran, Dimethoxyethan oder Dimethylsulfoxid in Gegenwart eines Säurefängers wie zum Beispiel Natriumhydroxid, Natriumhydrid oder Alkali- oder Erdalkalicarbonaten wie zum Beispiel Natrium-, Magnesium-, Kalium-, Calciumcarbonat bei Temperaturen zwischen 0 °C und dem Siedepunkt des jeweiligen Lösungsmittels, vorzugsweise jedoch zwischen 20 °C und 60 °C, durchgeführten Umsetzungen mit einem Substrat der allgemeinen Formel IV

Nf-L-Fu (IV),

worin Nf für ein Nucleofug wie z.B. Cl, Br, J, CH₃C₆H₄SO₃, oder CF₃SO₃, L für einen aliphatischen, aromatischen, arylaliphatischen, verzweigten, geradkettigen oder cyclischen Kohlenwasserstoffrest mit bis zu 20 Kohlenstoffatomen und Fu für die gewünschte endständige funktionelle Gruppe, gegebenenfalls in geschützter Form, stehen (DE-OS 34 17 413).

Als Beispiele für Verbindungen der allgemeinen Formel IV seien genannt Br(CH₂)₂NH₂, Br(CH₂)₃OH, BrCH₂COOCH₃, BrCH₂CO₂^{t}Bu, ClCH₂CONHNH₂,
Br(CH₂)₄CO₂C₂H₅, BrCH₂COBr, ClCH₂COOC₂H₅,
BrCH₂CONHNH₂,

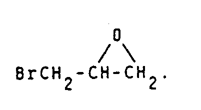

Umwandlungen von Carboxy-Gruppen können zum Beispiel nach der Carbodiimid-Methode (Fieser, Reagents for Organic Syntheses 10,142), über ein gemischtes Anhydrid [Org. Prep. Proc. Int. 7,215(1975)] oder über einen aktivierten Ester (Adv. Org. Chem. Part B, 472) durchgeführt werden.

Die Herstellung der als Ausgangssubstanzen für die Cyclisierung benötigten Amine erfolgt analog literaturbekannten Methoden.

Ausgehend von einer N-geschützten Aminosäure erhält man durch Umsetzung mit einem partiell geschützten Diamin (zum Beispiel nach der Carbodiimidmethode), Abspaltung der Schutzgruppen und Diboranreduktion ein Triamin.

Die Umsetzung eines aus Aminosäuren erhältlichen Diamins (Eur. J. Med. Chem.-Chim. Ther. 21,333 (1986)) mit der doppelten molaren Menge einer N-geschützten ω-Aminosäure liefert ein Tetramin nach geeigneter Aufarbeitung.

In beiden Fällen ist die Anzahl der Kohlenstoffatome zwischen den N-Atomen durch die Art der als Kopplungspartner eingesetzten Diamine bzw. Aminosäuren bestimmbar.

Die Einführung der gewünschten Metalle (z.B Mn, Fe, Co, Ni, Cu, Zn, Ga, In, Tc, Sn, Sm, Eu, Gd, Tl) in die Porphyrinliganden erfolgt nach literaturbekannten Methoden (The Porphyrins, ed. D. Dolphin, Academic Press, New York 1980, Vol. V, p. 459) durch Erwärmen mit den entsprechenden Metallsalzen, vorzugsweise den Acetaten, gegebenenfalls unter Zusatz von säurepuffernden Mitteln wie Natriumacetat. Als Lösungsmittel sind vor allem polare Solventien, wie zum Beispiel Chloroform, Essigsäure, Methanol, Ethanol, Dimethylformamid, Wasser geeignet.

Die Komplexierung sollte möglichst unter Lichtausschluß vorgenommen werden, da ein photochemischer Abbau der Porphyrine erfolgen kann.
Bevorzugte Metalle sind Mangan, Eisen, Technetium, Gallium und Indium.
Enthält die erfindungsgemäße Komplexverbindung den Rest K, so kann die Einführung des Porphyrin-Metalls vor oder nach Anknüpfung des Komplexbildner-Restes K sowie auch vor oder nach Chelatisierung dieses Komplexbildners mit einem oder mehreren Metall(en) erfolgen. Dadurch wird eine besonders flexible Vorgehensweise für die Synthese der erfindungsgemäßen Verbindungen ermöglicht, so daß zum Beispiel Metalle mit geringer Halbwertszeit, zum Beispiel ¹¹¹In, sei es in den Porphyrinliganden oder in den Komplexbildner, erst im letzten Syntheseschritt eingeführt werden können.
Die Chelatisierung des Restes K erfolgt in der Weise, wie sie in der Patentschrift DE-OS 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21-32, 37-39, 42-44, 49, 57-83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene azide Wasserstoffatome der Säuregruppen durch Kationen anorganischer und/oder organischer Basen oder Aminosäuren substituiert.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl-und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Säuregruppen im Komplex ganz oder teilweise in zum Beispiel Ester oder Amide zu überführen. Dies kann durch nachträgliche Reaktion am fertigen Komplex geschehen (z.B. durch erschöpfende Umsetzung der freien Carboxy-Gruppen mit Dimethylsulfat) wie auch durch Verwendung eines geeignet derivatisierten Substrats zur Einführung der Komplexbildner-Einheiten K-V² der allgemeinen Formeln I'A, I'B, I'C, I'D, I''AB, I''C, I''D (z.B. 3-Ethoxy-carbonylmethyl-6-[2-(2,6-dioxomorpholino-ethyl]-3,6-diazaoctandisäure.

Im Falle der Verwendung von Radioisotope enthaltenden Komplexverbindungen kann deren Herstellung nach den in "Radiotracers for Medical Applications", Volume 1 CRC-Press, Boca Raton, Florida beschriebenen Methoden vorgenommen werden.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.

Sind für die enterale Veratreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)} Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelattildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen wahrend des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 70µMol/L bis 70 m mol/L in Form seines Komplexsalzes und werden in der Regel in Mengen von 0,1 µMol bis 1 mMol/kg Körpergewicht dosiert. Sie sind zur enteralen und parenteralen Applikation bestimmt.

Die erfindungsgemäßen Komplexverbindungen kommen zur Anwendung
1. für die NMR- und Röntgen-Diagnostik in Form ihrer Komplexe mit den Ionen der Elemente mit den Ordnungszahlen 21-29, 42, 44 und 57-83;
2. für die Radiodiagnostik in Form ihrer Komplexe mit den Isotopen der Elemente mit den Ordnungszahlen 27, 29-32, 37-39, 43, 49, 62, 64, 70 und 77.

Die erfindungsgemäßen Mittel erfüllen die vielfältigen Voraussetzungen für die Eignung als Kontrastmittel für die Kernspintomographie. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es, hochkonzentrierte Lösungen herzustellen, damit die Volumenbelastung des Kreislaufs in vertretbaren Grenzen zu halten und die Verdünnung durch Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht konvalent gebundenen - an sich giftigen -Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 5 µMol bis 100 µMol/kg Körpergewicht, vorzugsweise 50 µMol bis 100 µMol/kg Körpergewicht, dosiert. Details der Anwendung werden zum Beispiel in H. J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts- Reagenzien und als shift-Reagenzien für die in-vivo NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida beschrieben.

Eine weitere bildgebende Methode mit Radioisotopen ist die Positronen-Emissions-Tomographie, die positronenemittierende Isotope wie z.B. ^{⁴³}Sc, ^{⁴⁴}Sc, ⁵²Fe, ⁵⁵Co, ⁶⁸Ga und ⁸¹Rb verwendet. (W.D. Heis, M.E. Phelps, Position Emission Tomography of Brain, Springer Verlag, Heidelberg, New York 1983).

Die erfindungsgemäßen Mittel sind ebenfalls als Röntgenkontrastmittel geeignet, wobei besonders hervorzuheben ist, daß sie auch im Vergleich zu den bisher gebräuchlichen jodhaltigen Kontrastmitteln eine für die Diagnostik wesentlich günstigeren Pharmakokinetik erkennen lassen. Besonders wertvoll sind sie weiterhin wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 100 µMol bis 1 mMol/kg Körpergewicht, vorzugsweise 300 µMol bis 800 µMol/kg Körpergewicht, dosiert.

Details der Anwendung von Röntgenkontrastmitteln werden zum Beispiel in Barke Röntgenkontrastmittel. G. Thieme, Leipzig (1970) und P. Thurn, E. Bücheler, Einführung in die Röntgendiagnostik, G. Thieme, Stuttgart, New York (1977) diskutiert.

Insgesamt ist es gelungen, neue Komplexverbindungen zu synthetisieren, die neue Möglichkeiten in der diagnostischen und therapeutischen Medizin erschließen.

Die nachfolgenden Beispiele dienen der näheren Erläuterung des Erfindungsgegenstandes.

### BEISPIEL 1

a) N-(6-tert-Butoxycarbonylamino-hexyl)-hematoporphyrin-IX-13-amid und
b) N,N'-Bis(6-tert-butoxycarbonylamino-hexyl)-hematoporphyrin-IX-13,17-diamid
In 300 ml Dimethylformamid werden 1,80 g (3 mmol) Hematoporphyrin IX, 810 mg (6 mmol) 1-Hydroxybenzotriazol, 1,52 g (6 mmol) N-Boc-1,6-diaminohexan Hydrochlorid und 608 mg (6 mmol) Triethylamin gegeben. Unter Rühren und Abdeckung mit Argon wird auf -10°C gekühlt und mit 1,24 g (6 mmol) Dicyclohexylcarbodiimid versetzt. Man läßt die Lösung 1 Stunde bei der tiefen Temperatur rühren und dann sich auf Raumtemperatur erwarmen. Der Verlauf der Umsetzung wird dünnschichtchromatographisch verfolgt. Nach 3 Tagen sind nur noch Reste vom Ausgangsmaterial vorhanden. Die Lösung wird nun im Ölpumpenvakuum zur Trockne eingeengt. Der Rückstand wird in Dichlormethan/Aceton gelöst und mit gesättigter Natriumbicarbonatlösung gewaschen. Die organische Lösung wird über Natriumsulfat getrocknet und im Vakuum zur Trockne eingeengt. Der Rückstand wird durch Säulenchromatographie an Kieselgel mit Isopropanol/Methanol (0-100%) in die beiden Titelverbindungen getrennt. Das Diamid wird als erste Verbindung von der Säule eluiert.
a) Ausbeute: 0,78 g (32,6% dere Theorie)

| | | | | |
|---|---|---|---|---|
| Analyse: | Ber.: | C 67,82 | H 7,59 | N 10,54 |
| | Gef.: | C 67,69 | H 7,76 | N 10,40 |

b) Ausbeute: 1,68 g (56,3% der Theorie)

| | | | | |
|---|---|---|---|---|
| Analyse: | Ber.: | C 67,58 | H 8,30 | N 11,26 |
| | Gef.: | C 67,49 | H 8,41 | N 11,20 |

c) N-(6-Aminohexyl)-hematoporphyrin-IX-13-amid
In 7 ml 2 molarer Salzsäure in Eisessig werden 490 mg (0,6 mmol) des unter Beispiel 1a hergestellten Monoamids gelöst und eine Stunde bei Raumtemperatur unter Feuchtigkeitsausschluß gerührt. Im Dünnschichtchromatogramm war danach kein Ausgangsmaterial mehr nachweisbar. Man engt im Vakuum zur Trockne ein, nimmt in destilliertem Wasser auf, unterwirft der Gefriertrocknung und erhält so die Titelverbindung als Trihydrochlorid.
Ausbeute: 450 mg (93,0 % der Theorie)

| | | | | | |
|---|---|---|---|---|---|
| Analyse: | Ber.: | C 57,74 | H 6,66 | N 10,10 | Cl 12,78 |
| | Gef.: | C 57,59 | H 6,83 | N 9,88 | Cl 12,82 |

### BEISPIEL 2

### N,N'-Bis(6-Aminohexyl)-hematoporphyrin-IX-13,17-diamid

In 8 ml 2 molarer Salzsäure in Eisessig werden 500 mg (0,5 mmol) des unter Beispiel 1b hergestellten Diamids gelöst und in Analogie zu der Vorschrift für Beispiel 1c behandelt.
Ausbeute: 400 mg (85% der Theorie)

| | | | | | |
|---|---|---|---|---|---|
| Analyse: | Ber.: | C 58,22 | H 7,50 | N 11,91 | Cl 15,07 |
| | Gef.: | C 57,98 | H 7,63 | N 11,73 | Cl 15,26 |

### BEISPIEL 3

a) N-(2-tert-Butoxycarbonylamino-ethyl)-hematoporphyrin-IX-13-amid
b) N,N'-Bis(2-tert-butoxycarbonylamino-ethyl)-hematoporphyrin-IX-13,17-diamid
In 250 ml Dimethylformamid werden 1,20 g (2 mmol) Hematoporphyrin-IX, 540,5 mg (4 mmol) 1-Hydroxybenzotriazol, 576,9 mg (4 mmol) N-(2-Aminoethyl)-carbaminsäure-tert-butylester sowie 825,3 mg (4 mmol) Dicyclohexylcarbodiimid wie im Beispiel 1 beschrieben umgesetzt. Man arbeitet dann analog dieser Vorschrift weiter. Das Diamid b) wird als unpolarere Verbindung zuerst eluiert. 457 mg (30,9% der Theorie) der Titelverbindung a)

| | | | | |
|---|---|---|---|---|
| Analyse: | Ber.: | C 66,47 | H 7,07 | N 11,34 |
| | Gef.: | C 66,32 | H 7,25 | N 11,24 |

967,9 mg (54,8% der Theorie) der Titelverbindung b)

| | | | | |
|---|---|---|---|---|
| Analyse: | Ber.: | C 65,28 | H 7,53 | N 12,69 |
| | Gef.: | C 65,02 | H 7,80 | N 12,47 |

c) N-(2-Aminoethyl)-hematoporphyrin-IX-13-amid
In 10 ml 2 molarer Salzsäure in Eisessig werden 741 mg (1 mmol) des unter Beispiel 3a beschriebenen Monoamids gelöst und analog Beispiel 1c umgesetzt und aufgearbeitet.
Ausbeute: 647,9 mg (86,6% der Theorie)

| | | | | | |
|---|---|---|---|---|---|
| Analyse: | Ber.: | C 57,80 | H 6,06 | N 11,23 | Cl 14,22 |
| | Gef.: | C 57,66 | H 6,18 | N 11,14 | Cl 14,34 |

### BEISPIEL 4

### N-(2-Aminoethyl)-3,8-bis(2-hydroxyethyl)-deuteroporphyrin-13-amid

Analog der Vorschrift für Beispiel 3a und 3c erhält man aus 3,8-Bis(2-hydroxyethyl)-deuteroporphyrin und N-(2-Aminoethyl)-carbaminsäure-t-butylester und anschließender Abspaltung der Boc-Schutzgruppe die Titelverbindung als Trihydrochlorid.

| | | | | | |
|---|---|---|---|---|---|
| Analyse: | Ber.: | C 57,80 | H 6,06 | N 11,23 | Cl 14,22 |
| | Gef.: | C 57,46 | H 5,91 | N 11,10 | Cl 14,43 |

### BEISPIEL 5

### N,N'-Bis(2-aminoethyl)-hematoporphyrin-IX-13,17-diamid

In 12 ml 2 molarer Salzsäure in Eisessig werden 883 mg ( 1 mmol) der Verbindung nach Beispiel 3b gelöst und analog Beispiel 1c umgesetzt und aufgearbeitet. Man erhält so eine Ausbeute von 701 mg (84,8% der Theorie) der Titelverbindung als Tetrahydrochlorid.

| | | | | | |
|---|---|---|---|---|---|
| Analyse: | Ber.: | C 55,21 | H 6,34 | N 13,55 | Cl 17,15 |
| | Gef.: | C 55,06 | H 6,48 | N 13,44 | Cl 17,29 |

### Alternative Darstellung:

α) N-(2-Benzyloxycarbonylamino-ethyl)-hematoporphyrin-IX-13-amid
ß) N,N'-Bis(2-benzyloxycarbonylamino-ethyl)-hematoporphyrin-IX-13,17-diamid
In 300 ml Dimethylformamid werden 1,80 g (3 mmol) Hematoporphyrin-IX, 810 mg (6 mmol) 1-Hydroxybenzotriazol, 1,38 g (6 mmol) N-(2-Aminoethyl)-carbaminsäurebenzylester-Hydrochlorid, 608 mg (6 mmol) Triethylamin und 1,24 g (6 mmol) Dicyclohexylcarbodiimid wie unter Beispiel 1 beschrieben umgesetzt und aufgearbeitet. Das Rohprodukt wird durch Säulenchromatographie an Kieselgel mit einem Gemisch aus Ethanol mit Ammoniak (25%) im Verhältnis 98/2 in die Komponenten getrennt. Das Diamid wird als erste Verbindung von der Säule eluiert.
α) Ausbeute: 0,52 g (22,4% der Theorie)

| | | | | |
|---|---|---|---|---|
| Analyse: | Ber.: | C 68,20 | H 6,50 | N 10,84 |
| | Gef.: | C 67,97 | H 6,72 | N 10,69 |

β) Ausbeute: 1,81 g (63% der Theorie)

| | | | | |
|---|---|---|---|---|
| Analyse: | Ber.: | C 68,19 | H 6,57 | N 11,78 |
| | Gef.: | C 67,85 | H 6,78 | N 11,61 |

δ) N,N'-Bis(2-aminoethyl)-hematoporphyrin-IX-13,17-diamid

In 6 ml Trifluoressigsäure werden 599 mg (0,63 mmol) des nach β) hergestellten Diamids gelöst und 12 Stunden bei Raumtemperatur unter Feuchtigkeitsausschluß gerührt. Dann wird im Vakuum zur Trockne eingeengt. Der Rückstand wird in destilliertem Wasser gelöst und an Kieselgel chromatographiert. Das Produkt wird mit Isopropanol/wäßriger Ammoniaklösung eluiert. Die vereinigten Eluate werden eingeengt, filtriert, in Wasser aufgenommen und gefriergetrocknet. Man erhält 333 mg (77,4 % der Theorie) der Titelverbindung als freies Amin.

| | | | | |
|---|---|---|---|---|
| Analyse: | Ber.: | C 66,84 | H 7,38 | N 16,41 |
| | Gef.: | C 66,93 | H 7,30 | N 16,25 |

### BEISPIEL 6

### N,N'-Bis(2-aminoethyl)-3,8-bis(2-hydroxyethyl)-deuteroporphyrin-13,17-diamid

Analog der Vorschrift für Beispiel 3b und 5 erhält man aus 3,8-Bis(2-hydroxyethyl)-deuteroporphyrin und N-(2-Aminoethyl)-carbaminsäure-t-butylester und anschließender Abspaltung der Boc-Schutzgruppen die Titelverbindung als Tetrahydrochlorid.

| | | | | | |
|---|---|---|---|---|---|
| Analyse: | Ber.: | C 55,21 | H 6,34 | N 13,55 | Cl 17,15 |
| | Gef.: | C 55,41 | H 6,29 | N 13,67 | Cl 17,03 |

### BEISPIEL 7

a) 5,0 g (35,6 mmol) D,L-2,3-Diaminopropionsäuremonohydrochlorid werden in 27 ml Wasser suspendiert, mit 72 ml tert.-Butanol versetzt und durch Zugabe von 37 ml 0,5 n Natriumcarbonatlösung auf pH 8,5 gestellt. In die klare Lösung tropft man bei pH 8,5 bis 9 gleichzeitig 32 g (142 mmol) Di-tert-butyldicarbonat und 114 ml 0,5 n Natriumcarbonatlösung. Nach Rühren über Nacht extrahiert man erschöpfend mit Hexan, bringt die wäßrige Phase durch Zugabe von 50 g Zitronensäure auf pH 4 und extrahiert sie erschöpfend mit Essigsäureethylester. Man trocknet über Natriumsulfat, engt zur Trockne ein und kristallisiert den Rückstand aus Essigsäureethylester/Hexan um. Man erhält 9,0 g (83% der Theorie) D,L-2,3-Bis(tert.-butoxycarbonylamino)propionsäure vom Fp.: 163-164° C
b) 3,04 g (10 mmol) der unter a) erhaltenen Verbindung werden in 50 ml Tetrahydrofuran gelöst. Unter Kühlung auf -12°C tropft man 1,21 g (12 mmol) Triethylamin und dann 1,66 g (12 mmol) Chlorameisensäureisobutylester unter Argon zu. Nach 20 Minuten nachrühren werden 2,00 g (18,67 mmol) Benzyloxycarbonylaminoethylamin zugesetzt und der Ansatz über Nacht stehen gelassen. Man saugt ab und engt das Filtrat im Vakuum zur Trockne ein. Der Rückstand wird in Methylenchlorid gelöst, die Lösung mit gesättigter Natriumbicarbonatlösung gewaschen und erneut zur Trockne eingeengt. Der Rückstand wird aus Essigsäureethylester/Hexan umkristallisiert. Man erhält 3,50 g (84% der Theorie) 2,3-Bis(tert-butoxycarbonylamino)-propionsäure(2-benzyloxycarbonylamino-ethyl)-amid vom Fp.: 152-154°C.
c) 2,02 g der unter b) erhaltenen Verbindung werden am Platinoxid-Katalysator drucklos hydriert. Nach dem Absaugen engt man das Filtrat zum öligen Rückstand ein. Man erhält 1,51 g (100% der Theorie) 2,3-Bis(tert-butoxycarbonylamino)propionsäure-(2-aminoethyl)-amid.

| | | | | |
|---|---|---|---|---|
| Analyse: | Ber.: | C 52,01 | H 8,73 | N 16,17 |
| | Gef.: | C 51,83 | H 8,54 | N 15,91 |

d)
α) N-{-2-[1,2-Bis(tert-butoxycarbonylamino)-ethyl-carbonylamino]-ethyl}-hematoporphyrin-IX-13-amid
β) N,N'-Bis{2-[1,2-bis(tert-butoxycarbonylamino)-ethyl-carbonylamino]-ethyl}-hematoporphyrin-IX-13,17-diamid
Unter den Bedingungen des Beispiels 1 werden 1,80 g Hematoporphyrin-IX, 810 mg (6 mmol) 1-Hydroxybenzotriazol, 2,079 g (6 mmol) der unter c) erhaltenen Verbindung in Gegenwart von 1,24 g (6 mmol) Dicyclohexylcarbodiimid umgesetzt. Das so erhaltene Gemisch wird durch Säulenchromatographie an Kieselgel mit Isopropanol/Methanol (0 - 100 %) in die Titelverbindungen getrennt. Das Diamid wird als erste Verbindung von der Säule eluiert. Beide Verbindungen werden als Schaum erhalten.
α) Ausbeute: 901 mg (32,4% der Theorie)

| | | | | | |
|---|---|---|---|---|---|
| Analyse: | Ber.: | C 63,48 | H 7,18 | N 12,09 | O 17,26 |
| | Gef.: | C 63,51 | H 7,26 | N 12,01 | |

ß) Ausbeute: 2,103 g (54,6% der Theorie)

| | | | | | |
|---|---|---|---|---|---|
| Analyse: | Ber.: | C 59,89 | H 7,38 | N 15,28 | O 17,45 |
| | Gef.: | C 59,78 | H 7,45 | N 15,22 | |

e) N-[2-(1,2-Diamino-ethylcarbonylamino)-ethyl]-hematoporphyrin-IX-13-amid
In Analogie zu Beispiel 1c werden 927 mg (1 mmol) des unter Beispiel dα hergestellten Monoamids in 15 ml 2 molarer Salzsäure in Eisessig gespalten und aufgearbeitet.
Man erhält so 752,3 mg der Titelverbindung (86,2% der Theorie).

| | | | | | | |
|---|---|---|---|---|---|---|
| Analyse: | Ber.: | C 53,67 | H 6,24 | N 12,84 | Cl 16,25 | O 11,00 |
| | Gef.: | C 53,56 | H 6,31 | N 12,75 | Cl 15,30 | |

### BEISPIEL 8

### N,N'-Bis[2-(1,2-diamino-ethylcarbonylamino)-ethyl]-hematoporphyrin-IX-13,17-diamid

Unter den Bedingungen des Beispiels 1c werden 1,284 g (1 mmol) des unter Beispiel 7dβ hergestellten Diamids in 20 ml 2, molarer Salzsäure in Eisessig gespalten und aufgearbeitet.

Man erhält so 919,2 mg der Titelverbindung (85,6% der Theorie).

| | | | | | | |
|---|---|---|---|---|---|---|
| Analyse: | Ber.: | C 49,22 | H 6,38 | N 15,65 | Cl 19,81 | O 8,94 |
| | Gef.: | C 49,28 | H 6,47 | N 15,60 | Cl 19,75 | |

### BEISPIEL 9

### N-(13-Carboxy-4-oxo-6,9,12-tris(carboxymethyl]-3,6,9,12-tetraazatridecyl)-hematoporphyrin-IX-13-amid

In 50 ml destilliertem Wasser werden 1,50 g (2 mmol) des nach Beispiel 3c hergestellten N-(2-Aminoethyl)-hematoporphyrin-IX-13-amid-Trihydrochlorids gelöst. Durch Zugabe von Natronlauge wird ein pH-Wert von 9 eingestellt. Zu dieser Losung gibt man bei 0°C unter Rühren anteilweise 887,4 mg 12,2 mmol) 3-Ethoxycarbonylmethyl-6-[2-(2,6-dioxomorpholino)ethyl]-3,6-diazaoctandisäure (DTPA-monoethylester-monoanhydrid), wobei man durch Natronlauge den pH-Wert zwischen 8-9 hält. Man läßt über Nacht bei Raumtemperatur bei einem pH-Wert von 10 stehen und reinigt den Komplexbildner durch Chromatographie an Kieselgel RP 18. Die Titelverbindung wird durch Methanol eluiert. Man engt die vereinigten Lösungen zur Trockne ein, nimmt den Rückstand in Wasser auf und unterwirft der Gefriertrocknung. Das Produkt wird als Schaum erhalten.
Ausbeute: 949,3 mg (84,3 % der Theorie)

| | | | | | | |
|---|---|---|---|---|---|---|
| Analyse: | Ber.: | C 53,33 | H 5,37 | N 11,20 | Na 10,21 | O 19,89 |
| | Gef.: | C 53,21 | H 5,47 | N 11,26 | Na 10,29 | |

### BEISPIEL 10

### N-(13-Carboxy-4-oxo-6,9,12-tris(carboxymethyl)-3,6,9,12-tetraazatridecyl)3,8-bis(2-hydroxyethyl)-deuteroporphyrin-13-amid

In analoger Weise erhält man aus der in Beispiel 4 erhaltenen Verbindung das entsprechende Deutheroporphyrin-Derivat.

| | | | | | | |
|---|---|---|---|---|---|---|
| Analyse: | Ber.: | C 53,33 | H 5,37 | N 11,20 | Na 10,21 | O 19,89 |
| | Gef.: | C 53,15 | H 5,42 | N 11,43 | Na 10,52 | |

### BEISPIEL 11

### N-(17-Carboxy-8-oxo-10,13,16-tris(carboxymethyl)-7,10,13,16-tetraazaheptadecyl) hematoporphyrin-IX-13-amid

Analog zu den Bedingungen des Beispiels 9 werden 806 mg (1 mmol) des nach Beispiel 1c hergestellten Trihydrochlorids mit 443,7 mg (1,1 mmol) DTPA-monoethylester-monoanhydrid umgesetzt. Man erhält nach Gefriertrocknung die Titelverbindung als Schaum.
Ausbeute: 1,031 g (87,2 % der Theorie)

| | | | | | | |
|---|---|---|---|---|---|---|
| Analyse: | Ber.: | C 54,87 | H 5,80 | N 10,66 | Na 9,72 | O 18,95 |
| | Gef.: | C 54,80 | H 5,87 | N 10,59 | Na 9,67 | |

### BEISPIEL 12

### N-{-5,6-Bis[9-carboxy-2,5,8-tris-(carboxymethyl)-2,5,8-triazanonylcarbonylamino]-4-oxo-3-azahexyl}-hematoporphyrin-IX-13-amid

Unter den Bedingungen des Beispiels 9 werden 872,7 mg (1 mmol) Diamin, hergestellt nach Beispiel 7e in 60 ml destilliertem Wasser mit 887,4 mg (2,2 mmol) DTPA-monoethylester-monoanhydrid umgesetzt. Aufarbeitung und Reinigung werden ebenfalls wie in Beispiel 11 durchgeführt. Die Titelverbindung wird nach Gefriertrocknung als Schaum erhalten.
Ausbeute: 1,409 g (84,1 % der Theorie)

| | | | | | | |
|---|---|---|---|---|---|---|
| Analyse: | Ber.: | C 48,03 | H 4,99 | N 11,70 | Na 12,35 | O 22,92 |
| | Gef.: | C 48,11 | H 5,07 | N 11,61 | Na 12,41 | |

### BEISPIEL 13

### N,N'-Bis{-5,6-bis[9-carboxy-2,5,8-tris-(carboxymethyl)-2,5,8-triazanonylcarbonylamino]-4-oxo-3-azahexyl}-hematoporphyrin-IX-13,17-diamid

In Analogie zu Beispiel 9 werden 1,074 g (1 mmol) des in Beispiel 8 hergestellten Tetraamins in 100 ml destilliertem Wasser gelöst und mit 1,775 g (4,4 mmol) DTPA-monoethylester-monoanhydrid umgesetzt. Aufarbeitung und Reinigung erfolgt ebenfalls wie unter Beispiel 9 beschrieben. Die Titelverbindung wird nach Gefriertrocknung als Schaum erhalten.
Ausbeute: 2,101 g (82,7% der Theorie)

| | | | | | | |
|---|---|---|---|---|---|---|
| Analyse: | Ber.: | C 47,28 | H 5,16 | N 13,23 | Na 14,48 | O 19,86 |
| | Gef.: | C 47,21 | H 5,24 | N 13,29 | Na 14,42 | |

### BEISPIEL 14

a) Hematoporphyrin-IX-mangan (III)-acetat, Dinatriumsalz
In einem Gemisch aus 45 ml Eisessig und 5 ml destilliertem Wasser werden 328 mg (4 mmol) wasserfreies Natriumacetat, 598,7 mg (1 mmol) Hematoporphyrin-IX und 981 mg (4 mmol) Mangan-(III)-acetat Tetrahydrat gelöst. Man erwärmt unter Rühren auf 50°C, wobei auf Lichtausschluß geachtet wird. Wenn dünnschichtchromatographisch kein Ausgangsmaterial mehr nachzuweisen ist, wird die Lösung im Vakuum zur Trockne eingeengt. Der Rückstand wird mit Wasser aufgeschlämmt und alkalisch gestellt. Man zentrifugiert, trennt den Feststoff ab, wäscht mit Wasser nach und entsalzt die vereinigten Lösungen durch Chromatographie an Kieselgel RP 18 (Merck LiChroprep RP 18). Das Produkt wird mit Methanol eluiert. Man engt die Lösung im Vakuum zur Trockne ein, nimmt in Wasser auf und unterwirft der Gefriertrocknung. Die Titelverbindung wird als Schaum erhalten.
Ausbeute: 659 mg (87,3% der Theorie)

| | | | | | | | |
|---|---|---|---|---|---|---|---|
| Analyse: | Ber.: | C 57,30 | H 4,94 | N 7,42 | Mn 7,28 | Na 6,09 | O 16,96 |
| | Gef.: | C 57,24 | H 5,01 | N 7,36 | Mn 7,22 | Na 6,10 | |

Der Mangangehalt wird mit dem Plasma Quad der Firma VG (England) bestimmt.
b) Mangan(III)-[N-(2-tert-butoxycarbonylamino-ethyl)-hematoporphyrin-IX-13-amid]-acetat
c) Mangan(III)-[N,N'-bis(2-tert-butoxycarbonylamino-ethyl)-hematoporphyrin-IX-13,17-diamid]-acetat
In einem Gemisch aus 40 ml Wasser und 20 ml Dioxan werden 2,264 g (3 mmol) des unter a) gewonnenen Mangankomplexes gelöst. Man versetzt mit 3 ml 1 molarer wäßriger Salzsäure, engt die Lösung im Vakuum zur Trockne ein, trocknet an der Ölpumpe und nimmt den Rückstand in 300 ml Dimethylformamid auf. Dann versetzt man mit 810 mg (6 mmol) 1-Hydroxybenzotriazol [Hydrat: 919 mg] und 961,3 mg (6 mmol) N-tert.-Butyloxycarbonyl-ethylendiamin. Man kühlt unter Rühren und Abdeckung mit Argon auf -10°C und versetzt mit 1,24 g (6 mmol) Dicyclohexylcarbodiimid. Man rührt eine Stunde bei dieser Temperatur und läßt dann auf Raumtemperatur kommen. Der Verlauf der Reaktion wird durch Dünnschichtchromatographie verfolgt. Nach 3 Tagen sind nur noch geringe Mengen Ausgangsmaterial erkennbar. Die Lösung wird nun im Ölpumpenvakuum zur Trockne eingeengt. Der Rückstand wird in Dichlormethan/Ethanol gelöst und mit gesättigter Kochsalzlösung gewaschen. Die organische Lösung wird über Natriumsulfat getrocknet und im Vakuum zur Trockne eingeengt. Der Rückstand wird der Säulenchromatographie an Kieselgel unterworfen. Mit Isopropanol/Methanol (0-100%) werden die Titelverbindungen getrennt. Die jeweiligen Lösungen werden vereinigt und im Vakuum zur Trockne eingeengt.
b) Ausbeute: 264 mg (30,9% der Theorie)

| | | | | | | |
|---|---|---|---|---|---|---|
| Analyse: | Ber.: | C 60,56 | H 6,26 | N 9,85 | Mn 6,44 | O 16,88 |
| | Gef.: | C 60,51 | H 6,30 | N 9,90 | Mn 6,39 | |

Der Mangangehalt wurde mit dem Plasma Quad bestimmt.
c) Ausbeute: 568 mg (57,1% der Theorie)

| | | | | | | |
|---|---|---|---|---|---|---|
| Analyse: | Ber.: | C 60,35 | H 6,79 | N 11,26 | Mn 5,52 | O 16,08 |
| | Gef.: | C 60,29 | H 6,85 | N 11,20 | Mn 5,47 | |

Der Mangangehalt wurde mit dem Plasma Quad bestimmt.
d) Mangan(III)-[N-(2-aminoethyl)-hematoporphyrin-IX-13-amid]-acetat
In 30 ml Trifluoressigsäure werden 853 mg (1 mmol) des nach Beispiel 14b erhaltenen Monoamids gelöst und bei Raumtemperatur gerührt. Nach etwa 15 Minuten ist kein Ausgangsmaterial mehr nachweisbar. Man zieht die Trifluoressigsäure im Vakuum ab, löst den Rückstand in destilliertem Wasser, stellt mit Ammoniak alkalisch, engt im Vakuum zur Trockne ein, löst in Wasser und unterwirft der Gefriertrocknung. Die Titelverbindung wird als Schaum erhalten.
Ausbeute: 659,4 mg (87,6% der Theorie)

| | | | | | | |
|---|---|---|---|---|---|---|
| Analyse: | Ber.: | C 60,63 | H 6,03 | N 11,16 | Mn 7,30 | O 14,88 |
| | Gef.: | C 60,57 | H 6,09 | N 11,08 | Mn 7,24 | |

Der Mangangehalt wurde mit dem ICP-Gerät bestimmt.
Dieselbe Verbindung wird durch Umsetzung der in Beispiel 3c beschriebenen Verbindung (Monoamin) mit Mangan-II-acetat analog Beispiel 14a erhalten.

### BEISPIEL 15

### Mangan(III)-N-(2-aminoethyl)-3,8-bis(2-hydroxyethyl)-deuteroporphyrin-17-amid-acetat

Die Titelverbindung wird durch Umsetzung der in Beispiel 4 beschriebenen Verbindung mit Mangan-II-acetat analog Beispiel 14a erhalten.

| | | | | | | |
|---|---|---|---|---|---|---|
| Analyse: | Ber.: | C 60,63 | H 6,03 | N 11,16 | Mn 7,30 | O 14,88 |
| | Gef.: | C 60,78 | H 6,01 | N 11,41 | Mn 7,15 | |

### BEISPIEL 16

### Mangan(III)-[N,N'-bis(2-aminoethyl)-hematoporphyrin-IX-13,17-diamid]-acetat

Analog zu den Bedingungen des Beispiels 14d werden 995 mg (1 mmol) des in Beispiel 14c dargestellten Bisamids mit Trifluoressigsäure gespalten. Man erhält nach Gefriertrocknung die Titelverbindung als Schaum.
Ausbeute: 681 mg (85,7% der Theorie)

| | | | | | | |
|---|---|---|---|---|---|---|
| Analyse: | Ber.: | C 60,45 | H 6,47 | N 14,10 | Mn 6,91 | O 12,08 |
| | Gef.: | C 60,52 | H 6,51 | N 14,06 | Mn 6,87 | |

Der Mangangehalt wird mit dem Plasma Quad bestimmt.

Dieselbe Verbindung wird durch Umsetzung der in Beispiel 5 beschriebenen Verbindung mit Mangan-II-acetat analog Beispiel 14a erhalten.

### BEISPIEL 17

a) Mangan(III)-[N-(6-tert-butoxycarbonylamino-hexyl)-hematoporphyrin-IX-13-amid]-acetat
b) Mangan(III)-[N,N'-bis(6-tert-butoxycarbonylamino-hexyl)-hematoporphyrin-IX-13,17-diamid]-acetat
Unter den Bedingungen des Beispiels 14a/b werden 2,264 g (3 mmol) des unter Beispiel 14a hergestellten Hematoporphyrin-IX-mangankomplexes in 300 ml Dimethylformamid mit 919 mg (6 mmol) 1-Hydroxybenzotriazol-Hydrat, 1,52 g (6 mmol) N-BOC-1,6-Diaminhexan Hydrochlorid, 608 mg (6 mmol) Triethylamin und 1,24 g (6 mmol) Dicyclohexylcarbodiimid umgesetzt. Aufarbeitung und Reinigung entsprechen dem Beispiel 14a/b.
a) Ausbeute: 278,2 mg (30,6% der Theorie)

| | | | | | | |
|---|---|---|---|---|---|---|
| Analyse: | Ber.: | C 62,11 | H 6,76 | N 9,25 | Mn 6,04 | O 15,84 |
| | Gef.: | C 62,01 | H 6,81 | N 9,18 | Mn 6,00 | |

Der Mangangehalt wird mit dem Plasma Quad bestimmt.
b) Ausbeute: 632,8 mg (60,2% der Theorie)

| | | | | | | |
|---|---|---|---|---|---|---|
| Analyse: | Ber.: | C 61,70 | H 7,19 | N 10,66 | Mn 5,23 | O 15,22 |
| | Gef.: | C 61,76 | H 7,15 | N 10,59 | Mn 5,19 | |

Der Mangangehalt wird mit dem Plasma Quad bestimmt.
c) Mangan(III)-[N-(6-aminohexyl)-hematoporphyrin-IX-13-amid]-acetat
Unter den Bedingungen des Beispiels 1c werden 909 mg (1 mmol) des unter Beispiel 17a hergestellten N-BOC-Derivats in 25 ml 2 molarer Salzsäure in Eisessig gespalten und ins Hydrochlorid überführt. Man erhält 738,8 mg der Titelverbindung (87,4% der Theorie).

| | | | | | | | |
|---|---|---|---|---|---|---|---|
| Analyse: | Ber.: | C 59,68 | H 6,44 | N 9,94 | Mn 6,50 | Cl 4,19 | O 13,25 |
| | Gef.: | C 59,61 | H 6,51 | N 9,87 | Mn 6,47 | Cl 4,23 | |

Dieselbe Verbindung wird durch Umsetzung der in Beispiel 1c beschriebenen Verbindung mit Mangan-II-acetat analog Beispiel 14a erhalten.

### BEISPIEL 18

### Mangan(III)-[N-(17-carboxy-8-oxo-10,13,16-tris-(carboxymethyl)-7,10,13,16-tetraazaheptadecyl)-hematoporphyrin-IX-13-amid]-acetat

In Analogie zu Beispiel 9 werden 845,3 mg (1 mmol) des unter Beispiel 17c hergestellten Amin-hydrochloridS in 50 ml destilliertem Wasser mit 443,7 mg (1,1 mmol) DTPA-monoethylester-monoanhydrid umgesetzt. Man erhält die Titelverbindung durch Gefriertrocknung als Schaum in Form des Pentanatriuzmsalzes.
Ausbeute: 1,102 g (85% der Theorie)

| | | | | | | | |
|---|---|---|---|---|---|---|---|
| Analyse: | Ber.: | C 52,01 | H 5,38 | N 9,75 | Na 8,82 | Mn 4,25 | O 19,79 |
| | Gef.: | C 51,96 | H 5,44 | N 9,80 | Na 8,76 | Mn 4,22 | |

Dieselbe Verbindung wird durch Umsetzung der in Beispiel 9 erhaltenen Verbindung mit Mangan-II-acetat analog Beispiel 14a erhalten. Ersetzt man Mangan-II-acetat durch Gallium-II-chlorid so erhält man den entsprechenden Galliumkomplex.

### BEISPIEL 19

### N-(13-Carboxy-4-oxo-6,9,12-tris-(carboxylatomethyl)-3,6,9,12-tetraazatridecyl)-hematoporphyrin-IX-13-amid, Gadolinium-Komplex

In 50 ml destilliertem Wasser werden 1,50 g (2 mmol) des nach Beispiel 3c hergestellten N-(2-Aminoethyl)-hemnatoporphyrin-IX-13-amid-Trihydrochlorids gelöst Durch Zugabe von Natronlauge wird ein pH-Wert von 9 eingestellt. Zu dieser Lösung gibt man bei 0°C unter Rühren anteilweise 806 mg (2 mmol) DTPA-monoethylester-monoanhydrid, wobei man durch Natronlauge den pH-Wert hält. Man läßt dann vier Stunden bei Raumtemperatur auf einen pH-Wert von 10 stehen, bringt dann mit verdünnter Salzsäure auf einen pH-Wert von 7,5 und versetzt nun anteilweise mit 813 mg (2 mmol) Gadoliniumacetat Tetrahydrat, wobei ebenfalls der pH-Wert durch Zusatz von Natronlauge gehalten wird. Man läßt über Nacht rühren, engt die Losung im Vakuum zur Trockne ein und reinigt das Produkt durch Chromatographie an Kieselgel RP 18. Die Lösung wird eingeengt, in Wasser aufgenommen und der Gefriertrocknung unterworfen.
Ausbeute: 1,95 g (80,3% der Theorie)

| | | | | | | | |
|---|---|---|---|---|---|---|---|
| Analyse: | Ber.: | C 49,46 | H 4,98 | N 10,38 | Na 3,79 | Gd 12,95 | O 18,45 |
| | Gef.: | C 49,38 | H 5,05 | N 10,29 | Na 3,69 | Gd 12,90 | |

Der Gadoliniumgehalt wird durch AAS bestimmt.

### BEISPIEL 20

### N-(13-Carboxy-4-oxo-6,9,12-tris-(carboxylatomethyl)-3,6,9,12-tetraazatridecyl)-3,8-bis(2-hydroxyethyl)-deuteroporphyrin-13-amid

Die isomere Verbindung wird analog zur Vorschrift für Beispiel 19 aus dem in Beispiel 10 beschriebenen Trihydrochlorid erhalten.

| | | | | | | | |
|---|---|---|---|---|---|---|---|
| Analyse: | Ber.: | C 49,46 | H 4,98 | N 10,38 | Na 3,79 | Gd 12,95 | O 18,45 |
| | Gef.: | C 49,71 | H 4,82 | N 10,53 | Na 3,59 | Gd 12,86 | |

In analoger Weise erhält man bei Verwendung von Dysprosiumacetat anstelle von Gadoliniumacetat die entsprechende Dysprosium-Komplexverbindung.

| | | | | | | | |
|---|---|---|---|---|---|---|---|
| Analyse: | Ber.: | C 49,24 | H 4,96 | N 10,34 | Na 3,77 | Dy 13,32 | O 18,37 |
| | Gef.: | C 49,13 | H 4,82 | N 10,81 | Na 3,51 | Dy 13,01 | |

### BEISPIEL 21

### N-(17-carboxy-8-oxo-10,13,16-tris-(carboxylatomethyl)-7,10,13,16-tetraazaheptadecyl)-hematoporphyrin-IX-13-amid, Gadolinium-Komplex

Analog zu den Bedingungen des Beispiels 19 werden 940 mg (1,2 mmol) des nach Beispiel 1c hergestellten 13-Monoamid-Trihydrochlorids, 580 mg (1,44 mmol) DTPA-monoethylester-monoanhydrid und 674 mg (1,44 mmol) Gadoliniumacetat Tetrahydrat in 100 ml destilliertem Wasser umgesetzt. Die Reinigung erfolgt durch Säulenchromatographie an Kieselgel RP 18, als Elutionsmittel dient Methanol.

| | | | | | | | |
|---|---|---|---|---|---|---|---|
| Analyse: | Ber.: | C 51,05 | H 5,40 | N 9,92 | Na 3,62 | Gd 12,38 | O 17,63 |
| | Gef.: | C 50,98 | H 5,47 | N 9,87 | Na 3,58 | Gd 12,30 | |

Die Gadoliniumbestimmung erfolgt durch AAS.

### BEISPIEL 22

### N-{5,6-Bis[9-carboxy-2,5,8-tris(carboxylatomethyl)-2,5,8-triazanonylcarbonylamino]-4-oxo-3-azahexyl}-hematoporphyrin-IX-14-amid, Di-Gd-Komplex

In 100 ml destilliertem Wasser werden 1,675 g (1 mmol) des unter Beispiel 12 hergestellten Komplexbildners gelöst und der pH-Wert wird auf 7,5 eingestellt. Zu der gewünschten Lösung werden anteilweise 812,9 mg (2 mmol) Gadoliniumacetat Tetrahydrat gegeben, wobei durch Zugabe von Natronlauge der pH-Wert konstant gehalten wird. Man läßt über Nacht nachrühren, reinigt den Komplex durch Säulenchromatographie an Kieselgel RP, engt die produkthaltigen Eluate im Vakuum zur Trockne ein, nimmt den Ruckstand in Wasser auf und gewinnt die Titelverbindung durch Gefriertrocknung.
Ausbeute: 1,615 g (87,2% der Theorie)

| | | | | | | | |
|---|---|---|---|---|---|---|---|
| Analyse: | Ber.: | C 43,45 | H 4,52 | N 10,59 | Na 3,72 | Gd 16,98 | O 20,73 |
| | Gef.: | C 43,39 | H 4,59 | N 10,52 | Na 3,64 | Gd 16,90 | |

Der Gadoliniumgehalt wird durch AAS bestimmt.

### BEISPIEL 23

### N,N'-Bis-{5,6-bis[9-carboxy-2,5,8-tris(carboxylatomethyl)-2,5,8-triazanonylcarbonylamino]-4-oxo-3-azahexyl}-hematoporphyrin-IX-13,17-diamid, Tetra-Gd-Komplex

Unter den Bedingungen des Beispiels 19 werden 1,2703 g (0,5 mmol) des unter Beispiel 12 hergestellten Komplexbildners in 100 ml destilliertem Wasser gelöst und mit 812,9 mg (2 mmol) Gadoliniumacetat Tetrahydrat umgesetzt. Aufarbeitung und Reinigung des Komplexbildners erfolgen gemäß Beispiel 19. Man erhält die Titelverbindung als Schaum.
Ausbeute: 1,185 g (81,9% der Theorie)

| | | | | | | | |
|---|---|---|---|---|---|---|---|
| Analyse: | Ber.: | C 41,51 | H 4,53 | N 11,62 | Na 3,18 | Gd 21,74 | O 17,43 |
| | Gef.: | C 41,46 | H 4,60 | N 11,57 | Na 3,11 | Gd 21,68 | |

Der Gadoliniumgehalt wird durch AAS bestimmt.

### BEISPIEL 24

a) N-[9,10-Bis(tert-butoxycarbonylamino)-8-oxo-7-azadecyl]-hematoporphyrin-IX-13-amid
In einem Gemisch aus 50 ml Wasser und 25 ml Dioxan werden 806 mg (1 mmol) des unter Beispiel 1c hergestellten Aminoderivats gelöst. Die Lösung wird mit Natronlauge auf einen pH-Wert von 8 gebracht. Man kühlt auf 4°C und tropft die Lösung von 401,4 mg (1 mmol) 2,3-Bis(tert-butoxycarbonylamino)-propionsäure-hydroxysuccinimidester dazu. Die Umsetzung wird dünnschichtchromatographisch verfolgt. Man engt im Vakuum zur Trockne ein, nimmt in Essigester auf und wäscht mit Natriumbicarbonatlösung, verdünnter Citronensäure und Kochsalzlösung, trocknet die organische Lösung über Natriumsulfat, engt sie im Vakuum ein und unterwirft den Rückstand der Säulenchromatographie an Kieselgel, wobei Isopropanol, dem Methanol in steigenden Mengen zugesetzt wird, als Elutionsmittel dient. Die Titelverbindung wird als Schaum erhalten.
Ausbeute: 676,5 mg (68,8% der Theorie)

| | | | | | |
|---|---|---|---|---|---|
| Analyse: | Ber.: | C 64,74 | H 7,59 | N 11,40 | O 16,27 |
| | Gef.: | C 64,68 | H 7,66 | N 11,43 | |

b) N-{9,10-Bis[9-carboxo-2,5,8-tris-(carboxymethyl)-2,5,8-triazanonylcarbonylamino]-8-oxo-7-azadecyl}-hematoporphyrin-IX-13-amid
In 20 ml 2 molarer Salzsäure in Eisessig werden 983,2 mg (1 mmol) des unter Beispiel 24a hergestellten Monoamids gelöst und eine Stunde bei Raumtemperatur unter Feuchtigkeitsausschluß gerührt. Im Dünnschichtchromatogramm ist danach kein Ausgangsmaterial mehr erkennbar. Man engt im Vakuum zur Trockne ein, nimmt den Rückstand in 50 ml destilliertem Wasser auf, stellt durch Zugabe von Natronlauge einen pH-Wert von 9 ein, kühlt auf 0°C und gibt nun unter Rühren anteilweise 887,4 mg (2,2 mmol) DTPA-monoethylester-monoanhydrid hinzu, wobei man durch Zugabe von Natronlauge den pH-Wert zwischen 8 -9 hält. Man läßt eine Stunde nachrühren, stellt die Lösung auf einen pH-Wert von 10 und läßt über Nacht stehen. Dann stumpft man mit Salzsäure auf einen pH-Wert von 7,5 ab und versetzt nun anteilweise unter Rühren mit 894,2 mg (2,2 mmol) Gadoliniumacetat Tetrahydrat, wobei ebenfalls der pH-Wert durch Zusatz von Natronlauge gehalten wird. Man läßt 4 Stunden nachrühren, engt die Lösung im Vakuum ein und reinigt das Produkt durch Säulenchromatographie an Kieselgel RP 18. Die Titelverbindung wird durch Gefriertrocknung gewonnen.
Ausbeute: 1,322 g (69,3% der Theorie)

| | | | | | | | |
|---|---|---|---|---|---|---|---|
| Analyse: | Ber.: | C 44,69 | H 4,81 | N 10,28 | Na 3,61 | Gd 16,48 | N 20,12 |
| | Gef.: | C 44,60 | H 4,86 | N 10,20 | Na 3,55 | Gd 16,39 | N 20,16 |

Der Gadoliniumgehalt wird durch AAS bestimmt.

### BEISPIEL 25

### N,N'-(17-carboxy-8-oxo-10,13,16-tris(carboxylatomethyl)-7,10,13,16-tetraazaheptadecyl)-hematoporphyrin-IX-13,17-diamid, Di-Gd-Komplex

Analog den Bedingungen des Beispiels 19 werden 615 mg (0,65 mmol) des 3,17-Diamids des Beispiels 2, 807 mg (2,0 mmol) DTPA-monoethylester-monoanhydrid und 937 mg (2,0 mmol) Gadoliniumacetat Tetrahydrat in 100 ml destilliertem Wasser umgesetzt. Die Reinigung erfolgt durch Säulenchromatographie an Kieselgel RP, als Elutionsmittel dient Methanol.
Ausbeute: 1,49 g (78,4% der Theorie)

| | | | | | | | |
|---|---|---|---|---|---|---|---|
| Analyse: | Ber.: | C 46,83 | H 5,31 | N 10,33 | Na 2,42 | Gd 16,57 | O 18,54 |
| | Gef.: | C 46,75 | H 5,40 | N 10,28 | Na 2,39 | Gd 16,43 | |

Der Gadoliniumgehalt wurde durch AAS bestimmt.

### BEISPIEL 26

### N,N'-Bis(13-carboxy-4-oxo-6,9,12-tris(carboxylatomethyl)-3,6,9,12-tetraazatridecyl)-hematoporphyrin-IX-13,17-diamid, Di-Gd-Komplex

In Analogie zu Beispiel 25 werden 413,4 mg (0,5 mmol) des Diamids des Beispiels 5, 807 mg (2.0 mmol) DTPA-monoethylester-monoanhydrid und 937 mg (2.0 mmol) Gadoliniumacetat Tetrahydrat in 100 ml destilliertem Wasser umgesetzt. Die Reinigung erfolgt durch Säulenchromatographie an Kieselgel RP. Die Titelverbindung wird durch Gefriertrocknung der wäßrigen Lösung erhalten.
Ausbeute: 707 mg (81,8% der Theorie)

| | | | | | | | |
|---|---|---|---|---|---|---|---|
| Analyse: | Ber.: | C 44,49 | H 4,55 | N 9,73 | Na 2,66 | Gd 18,20 | O 20,37 |
| | Gef.: | C 44,41 | H 4,60 | N 9,68 | Na 2,62 | Gd 18,15 | |

Der Gadoliniumgehalt wird durch AAS bestimmt.

### BEISPIEL 27

### Mangan(III)-[N-(17-carboxy-8-oxo-10,13,16-tris(carboxylatomethyl)-7,10,13,16-tetraazaheptadecyl)-hematoporphyrin-IX-13-amid]-acetat, Gadolinium-Komplex

909 mg (1 mmol) des unter Beispiel 17a hergestellten geschützten Monoamids werden mit Salzsäure in Eisessig wie unter Beispiel 17c beschrieben gespalten und dann in destilliertem Wasser mit 443,7 mg (1,1 mmol) DTPA-monoethylester-monoamid zum Komplexbildner und dann mit 447,1 mg Gadoliniumacetat Tetrahydrat nach der Vorschrift für Beispiel 19 zur Titelverbindung umgesetzt. Nach Gefriertrocknung der gereinigten Substanz erhält man 935,9 mg (67,7% der Theorie) als Schaum.

| | | | | | | | | |
|---|---|---|---|---|---|---|---|---|
| Analyse: | Ber.: | C 48,66 | H 5,03 | N 9,12 | Na 3,33 | Gd 11,38 | Mn 3,97 | O 18,52 |
| | Gef.: | C 48,59 | H 5,10 | N 9,14 | Na 3,36 | Gd 11,30 | Mn 3,94 | |

Der Metallgehalt wird mit dem Plasma Quad bestimmt.

### BEISPIEL 28

### Mangan(III)-[N-(13-carboxy-4-oxo-6,9,12-tris(carboxylatomethyl)-3,6,9,12-tetraazatridecyl)-hematoporphyrin-IX-13-amid]-acetat, Gadolinium-Komplex

In 30 ml Trifluoressigsäure werden 853 mg (1 mmol) des unter Beispiel 14b hergestellten geschützten Monoamids des Hematoporphyrinmangankomplexes gelöst und bei Raumtemperatur gerührt. Nach etwa 15 Minuten ist dünnschichtchromatographisch kein Ausgangsmaterial nachweisbar. Man engt im Vakuum zur Trockne ein, nimmt in 100 ml destilliertem Wasser auf, kühlt auf 0°C, stellt durch Zugabe von Natronlauge einen pH-Wert von 9 ein und gibt anteilweise unter Ruhren 443,4 mg (1,1 mmol) DTPA-monoethylester-monoanhydrid hinzu, wobei durch weitere Zugabe von Natronlauge der pH-Wert gehalten wird. Man läßt zwei Stunden nachrühren, stellt dann einen pH-Wert von 10 ein und läßt über Nacht stehen. Dann wird mit verdünnter Salzsäure auf einen pH-Wert von 7,5 abgestumpft und man versetzt nun anteilweise mit 417,1 mg (1,1 mmol) Gadoliniumacetat Tetrahydrat, wobei ebenfalls der pH-Wert durch Zugabe von Natronlauge gehalten wird. Man läßt über Nacht rühren und reinigt das Produkt durch Chromatographie an Kieselgel RP. Die produkthaltige Lösung wird im Vakuum zur Trockne eingeengt, in Wasser aufgenomnmen und gefriergetrocknet. Die Titelverbindung wird als Schaum erhalten. Ausbeute: 1,089 g (82,1% der Theorie)

| | | | | | | | | |
|---|---|---|---|---|---|---|---|---|
| Analyse: | Ber.: | C 47,09 | H 4,64 | N 9,50 | Na 3,47 | Mn 4,14 | Gd 11,86 | O 19,30 |
| | Gef.: | C 47,02 | H 4,70 | N 9,45 | Na 3,50 | Mn 4,10 | Gd 11,78 | |

Der Metallgehalt wird mit dem Plasma Quad bestimmt.

Die Verbindung wird auch auf folgende Weise erhalten:

In ein Gemisch aus 22 ml Eisessig und 2 ml destilliertem Wasser werden 123 mg (1,5 mmol) wasserfreies Natriumacetat gegeben und man erwärmt auf 50°C. Dann gibt man 380 mg (0,32 mmol) des unter Beispiel 19 hergestellten Gadoliniumkomplexes sowie 368 mg (1,5 mmol) Mangan-(II)-acetat Tetrahydrat zu und rührt unter Lichtausschluß bei dieser Temperatur etwa 2 Stunden. Im Dünnschichtchromatogramm ist kein Ausgangsmaterial mehr erkennbar. Man engt im Vakuum zur Trockne ein, nimmt in Wsser auf, filtriert und reinigt durch Chromatographie an Kieselgel RP. Die produkthaltigen Lösungen werden vereinigt, im Vakuum zur Trockne eingeengt, in Wasser aufgenommen und der Gefriertrocknung unterworfen. Man erhält so 357,8 mg (84,3% der Theorie) der Titelverbindung.

Mangan und Gadolinium werden mit dem Plasma Quad bestimmt.

| | | | | | | | | |
|---|---|---|---|---|---|---|---|---|
| Analyse: | Ber.: | C 47,09 | H 4,64 | N 9,50 | Na 3,47 | Mn 4,14 | Gd 11,86 | O 19,30 |
| | Gef.: | C 47,02 | H 4,71 | N 9,46 | Na 3,43 | Mn 4,09 | Gd 11,80 | |

### BEISPIEL 29

### Mangan(III)-[N,N'-bis(13-carboxy-4-oxo-6,9,12-tris(carboxylatomethyl)-3,6,9,12-tetraazatridecyl)-hematoporphyrin-IX-13,17-diamid]-acetat, Di-Gadolinium-Komplex

Die nach Abspaltung der Stickstoff-Schutzgruppen aus 995 mg (1,0 mmol) Edukt erhaltene Substanz des Beispiels 16 wird in 100 ml destilliertem Wasser mit 887,4 mg (2,2 mmol) DTPA-monoethylester-monoanhydrid sowie 894 mg (2,2 mmol) Gadoliniumacetat Tetrahydrat analog Beispiel 19 umgesetzt und gereinigt. Die Titelverbindung erhält man als Schaum. Ausbeute: 1,562 g (82,3% der Theorie)

| | | | | | | | | |
|---|---|---|---|---|---|---|---|---|
| Analyse: | Ber.: | C 43,03 | H 4,51 | N 10,33 | Na 2,42 | Mn 2,89 | Gd 16,57 | O 20,23 |
| | Gef.: | C 42,97 | H 4,57 | N 10,28 | Na 2,38 | Mn 2,86 | Gd 16,49 | |

Die Metallbestimmung wird mit dem Plasma Quad vorgenommen.

Dieselbe Verbindung wird auch auf folgende Weise erhalten:
In Analogie zu Beispiel 44a werden 1037 mg (0,6 mmol) des unter Beispiel 26 hergestellten Digadoliniumkomplexes zu der 50°C warmen Lösung von 197 mg (2,4 mmol) wasserfreiem Natriumacetat in einem Gemisch aus 45 ml Eisessig und 5 ml destilliertem Wasser gegeben. Dann fügt man 588 mg (2,4 mmol) Mangan-(II)-acetat Tetrahydrat hinzu und rührt unter Lichtausschluß bis im Dünnschichtchromatogramm kein Ausgangsmaterial erkennbar ist. Nach Aufarbeitung erhält man 906,5 mg (79,6% der Theorie) der Titelverbindung.

Mangan und Gadolinium werden mit dem Plasma Quad bestimmt.

| | | | | | | | | |
|---|---|---|---|---|---|---|---|---|
| Analyse: | Ber.: | C 43,03 | H 4,51 | N 10,33 | Na 2,42 | Mn 2,89 | Gd 16,57 | O 20,23 |
| | Gef.: | C 42,98 | H 4,60 | N 10,28 | Na 2,37 | Mn 2,87 | Gd 16,52 | |

### BEISPIEL 30

### Di-Mangan-III-{N,N'-[4,14-dioxo-6,9,12-tris(carboxylatomethyl)-3,6,9,12,15-pentaaza-1,17-heptadecylmethylen]-bis(hematoporphyrin)-IX-13-amid}-diacetat, Pentanatriumsalz

1,21 g (1,61 mmol) Mangan-III-[N-(2-Aminoethyl)-hematoporphyrin-IX-13amid)-acetat (Beispiel 14d) werden in 35 ml Wasser durch Zugabe von n-Natronlauge bei pH 10 gelöst. Die Lösung wird bei 0°C portionsweise mit 0,29 g (0,805 mmol) 1,5Bis(2,6-dioxomorpholino)-3-azapentan-3-essigsäure versetzt, wobei man den pH-Wert durch Zutropfen von n-Natronlauge auf 9,5 bis 10 hält. Man läßt drei Stunden bei Raumtemperatur nachrühren und engt die Lösung zur Trockne ein. Der Rückstand wird in 100 ml Eisessig gelöst und die Lösung nach Zugabe von 394 mg (1,61 mmol) Mangan-II-acetat-tetrahydrat drei Stunden auf 50°C erwärmt. Man engt die Lösung zur Trockne ein, nimmt den Rückstand in Wasser auf und chromatographiert die Lösung an Kieselgel. Nach Gefriertrocknung erhält man 1,56 g (79 % der Theorie) der Titelverbindung als weißes Pulver.

| | | | | | |
|---|---|---|---|---|---|
| Analyse: | Ber.: | C 54,80 | H 5,31 | N 10,65 | Mn 5,57 |
| | Gef.: | C 54,63 | H 5,52 | N 10,33 | Mn 5,40 (wasserfreie Substanz) |

### BEISPIEL 31

### Herstellung eines Kontrastmittels für die nuclearmedizinische Anwendung mit ¹¹¹In

2,252 mg (2 µmol) der in Beispiel 9 beschriebenen Verbindung werden in 3 ml sterilem, pyrogenfreiem 0,1 m Citratpuffer (pH 5,8) gelöst. Die Lösung wird über ein Millipore-Filter (0,2 µm) in ein Multivial filtriet und mit 0,1 ml einer physiologischen Kochsalzlösung, enthaltend 1,3 mCi ¹¹¹In Cl₃ versetzt. Die Lösung ist gebrauchsfertig.

In analoger Weise erhält man aus dem in Beispiel 18 aufgeführten Galliumkomplex das entsprechende ¹¹¹In-Radiodiagnostikum.

### BEISPIEL 32

### Herstellung eines Kontrastmittels für die nuclearmedizinische Anwendung mit ^{99m}TC

1,689 mg (1,5 µmol) der in Beispiel 9 beschriebenen Verbindung werden in 1,5 ml sterilem, destilliertem Wasser gelöst und mit 0,26 mg Natriumhydrogensulfit (1,5 µmol) versetzt. Nach Zugabe von 0,1 n-Salzsäure bis pH 5,8 wird die Lösung über ein Millipore-Filter (0,2 µm) gegeben und in ein Multivial unter Stickstoffbegasung gefüllt. Nach Zugabe von 0,5 ml einer physiologischen Kochsalzlösung, die 5,0 mCi Technetium (^{99m}Tc)-Pertechnetat enthält, erhält man eine gebrauchsfertige Lösung.

### BEISPIEL 33

### Herstellung eines Kontrastmittels für die NMR-Diagnostik

46,4 g (35 mmol) der in Beispiel 28 beschriebenen Verbindung werden in 500 ml einer sterilen und pyrogenfreien Pufferlösung (0.1 m Natriumhydrogencarbonat) unter Zusatz von 500 mg des Calcium-Trinatrium-Salzes der Diethylentriaminpenta essigsäure und unter Einleiten von Kohlendioxid neutral gelöst. Die Lösung wird steril filtriert und in 50 ml-Portionen in Flaschen abgefüllt.

### BEISPIEL 34

### Herstellung eines Kontrastmittels für die NMR-Diagnostik

45,29 g (35 mmol) der in Beispiel 18 beschriebenen Verbindung werden in 500 ml einer sterilen und pyrogenfreien Pufferlösung (0,1 m Natriumhydrogencarbonat) unter Zusatz von 500 mg des Calcium-Dinatrium-Salzes der Diethylentriaminpentaessigsäure und durch Begasung mit Kohlendioxid neutral gelöst. Die steril filtrierte Lösung wird in 50 ml-Portionen in Multivials abgefüllt.

### BEISPIEL 35

### Herstellung eines Kontrastmittels für die NMR-Diagnostik

42,49 g (35 mmol) der in Beispiel 19 beschriebenen Verbindung werden in 500 ml einer sterilen und pyrogenfreien Pufferlösung (0,1 m Natriumhydrogencarbonat) unter Zusatz von 500 mg des Calcium-Trinatrium-Salzes der Diethylentriaminpentaessigsäure und durch Begasung mit Kohlendioxid neutral gelöst. Die steril filtrierte Lösung wird in 50 ml-Portionen in Flaschen abgefüllt.

### BEISPIEL 36

### Herstellung eines Kontrastmittels für die NMR-Diagnostik

691,2 g (0,4 mol) der in Beispiel 26 beschriebenen Verbindung werden in 500 ml einer sterilen und pyrogenfreien Pufferlösung (0,1 m Natriumhydrogencarbonat) unter Zusatz von 500 mg des Calcium-Dinatrium-Salzes der Diethylentriaminpentaessigsäure und durch Begasung mit Kohlendioxid neutral unter Erwärmen gelöst. Die steril filtrierte Lösung wird in 50 ml-Portionen in Flaschen abgefüllt.

### BEISPIEL 37

a) 3,8-Bis-(1-methoxyethyl)-deuteroporphyrin-13,17-dihydrazid
In 20 ml Pyridin werden 1,0 g (1,53 mmol) Hematoporphyrin-IX-dimethylester-dimethylether (S. Kojo und S. Sano, J . Chem. Soc. Perkin I, 2864, 1981) gelöst, mit 6 ml Hydrazinhydrat (80 %) versetzt und über Nacht bei Raumtemperatur gerührt. Im Dünnschichtchromatogramm ist kein Ausgangsmaterial erkennbar. Die Lösung wird im Vakuum zur Trockne eingeengt. Die Titelverbindung wird durch Säulenchromatographie an Kieselgel mit Ethanol als Elutionsmittel gereinigt. Nach Trocknen im Vakuum erhält man das Produkt als Schaum.
Ausbeute: 0,73 g (72,9 % der Theorie)

| | | | | |
|---|---|---|---|---|
| Analyse: | Ber.: | C 66,03 | H 7,08 | N 17,11 |
| | Gef.: | C 66,24 | H 6,95 | N 16,95 |

b) N,N'-Bis[10-ethoxycarbonyl-3,6,9-tris(carboxylatomethyl)-3,6,9-triazadecanoylamino]-3,8-bis(1-methoxyethyl)deuteroporphyrin-13,17-diamid, Gd-Komplex
In einem Gemisch aus 60 ml Dioxan und 40 ml destilliertem Wasser werden 654,8 mg (1 mmol) des nach Beispiel 39a) hergestellten Dihydrazids gelöst. Man setzt dann bei 0 °C unter Rühren mit 1,211 g (3,0 mmol) 3-Ethoxycarbonylmethyl-6-[2-(2,6-dioxomorpholino)ethyl]-3,6-diazaoctandisäure um, wobei man den pH-Wert der Lösung durch Zugabe von Natronlauge zwischen 8 - 9 hält. Die Lösung wird 2 Stunden nachgerührt. Man stellt dann den pH-Wert der Lösung mit verdünnter Salzsäure auf 7,5 ein und gibt anschließend anteilweise 1,219 g (3 mmol) Gadoliniumacetat Tetrahydrat dazu, wobei ebenfalls der pH-Wert durch Zugabe von Natronlauge gehalten wird. Nach Rühren über Nacht wird im Vakuum zur Trockne eingeengt. Das Produkt wird durch Chromatographie an Kieselgel RP 18 gereinigt. Die die Titelverbinduöng enthaltende Lösung wird im Vakuum zur Trockne eingeengt, in destilliertem Wasser aufgenommen und der Gefriertrocknung unterworfen.
Ausbeute: 1,347 g (76,1 % der Theorie)

| | | | | | | |
|---|---|---|---|---|---|---|
| Analyse: | Ber.: | C 46,14 | H 5,13 | N 11,08 | Gd 17,77 | O 19,89 |
| | Gef.: | C 46,02 | H 5,24 | N 11,17 | Gd 17,61 | |

### BEISPIEL FÜR DIE NMR-DIAGNOSTIK IN VIVO

Der Gadoliniumkomplex des Mangan-(III)-[N-(13-carboxy-4-oxo-6,9,12-tris(carboxylatomethyl)-3,6,9,12-tetraazatridecyl)-hematoporphyrin-IX-13-amid]-acetat, Dinatriumsalz (Beispiel 28) wurde in einer Dosierung von 0,1 mmol/kg einer Nacktmaus (Balb/c, nu/nu, = 20g) mit einem subcutanen HT29 Colontumor i.v. appliziert. Nach Lösung der Substanz wurde ein pH-Wert von 7,5 eingestellt. Die Untersuchung wurde in einem Kernspintomographen (Firma General Electric) mit einem 2 Tesla Magneten durchgeführt.

Es wurden Aufnahmen vor und nach der Applikation des Kontrastmittels (T_{R} = 400 msec. T_{E} = 30 msec) im Bereich der Leber und des Tumors gemacht.

## Patentansprüche

1. Porphyrin-Komplexverbindungen, bestehend aus mindestens einem Ion eines Elements der Ordnungszahlen 5, 13, 21-32, 37-39, 42-44, 49, 50 oder 57-83 und mindestens einem Porphyrinliganden der allgemeinen Formel I 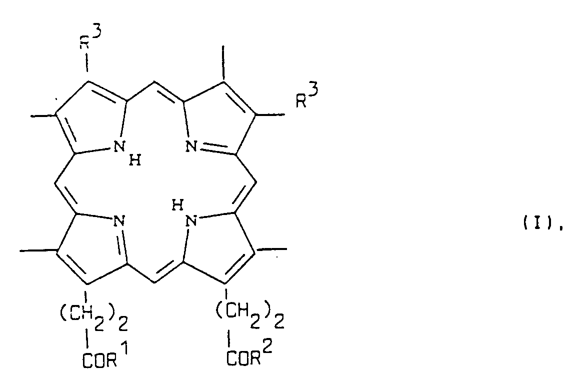 worin
R¹ und R² unabhängig voneinander für die OH- oder (NH)ₓ-[Q-(NH)_{y}]_{w}-W-Gruppe,
wobei
X und y die Ziffern 0, 1 oder 2,
w die Ziffern 0 oder 1,
Q eine C₀-C₂₀-Alkylengruppe,
W ein Wasserstoffatom oder die Gruppierung V-K bedeuten, wobei
V eine gegebenenfalls Imino-, Polyethylenoxy-, Phenylenoxy-, Phenylenimino-, Amido-, Hydrazido-, Estergruppe(n), Sauerstoff-, Schwefel-und/oder Stickstoff-Atom(e) enthaltende gegebenenfalls durch Hydroxy-, Mercapto-, Imino-, Epoxy-, Oxo-, Thioxo- und/oder NHK-gruppe(n) substituierte geradkettige, verzweigte, gesättigte oder ungesättigte C₀-C₂₀-Alkylengruppen,
K ein Wasserstoffatom oder ein Komplexbildner der allgemeinen Formeln IA, IB, IC oder ID 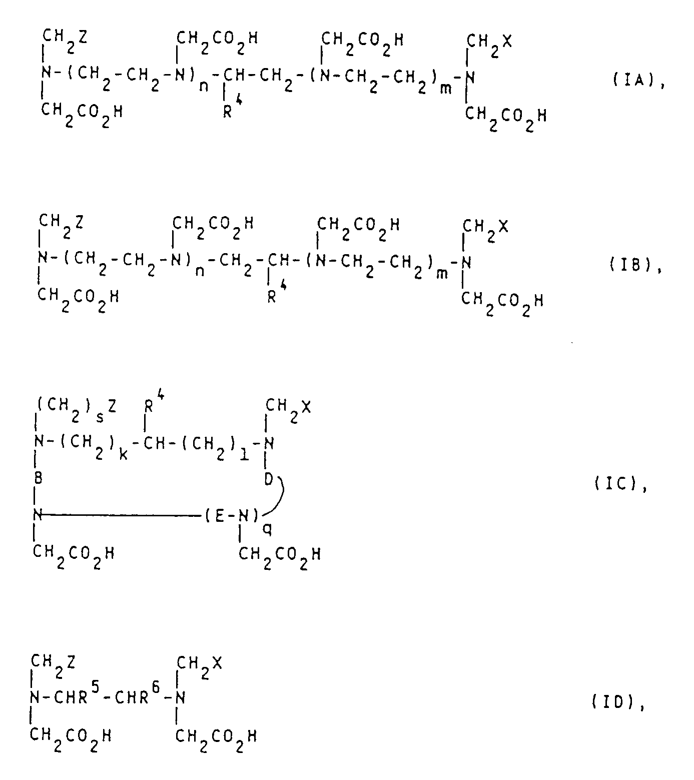 bedeuten,
mit der Maßgabe, daß entweder Z oder R⁴ an V gebunden ist,
wobei
n und m jeweils für die Ziffern 0, 1, 2, 3 oder 4, wobei n und m zusammen nicht mehr als 4 ergeben,
k für die Ziffern 1, 2, 3, 4 oder 5,
l für die Ziffern 0, 1, 2, 3, 4 oder 5,
q für die Ziffern 0, 1 oder 2,
s für die Ziffern 0 oder 1,
B, D und E, die gleich oder verschieden sind, jeweils für die Gruppe 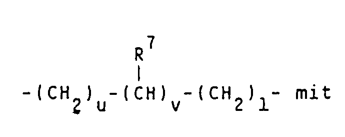
R⁷ in der Bedeutung von Wasserstoff oder einer gegebenenfalls Sauerstoff- und/oder Stickstoffatom(e) enthaltenden gegebenenfalls durch Hydroxy-und/oder Aminogruppe(n) substituierten geradkettigen, verzweigten, gesättigten oder ungesättigten C₁-C₂₀-Alkylgruppe,
u in der Bedeutung der Ziffern 0, 1, 2, 3, 4 oder 5,
v in der Bedeutung der Ziffern 0 oder 1, wobei B, D und E jeweils mindestens 2 und maximal 5 Kohlenstoffatome enthalten,
Z für den Rest -CO₂H oder die Gruppe 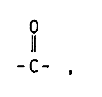
X für den Rest -CO₂H oder 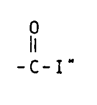 mit I'' in der Bedeutung eines weiteren Porphyrinliganden der allgemeinen Formel I, wobei der in R¹ und R² enthaltene Rest W hier für eine direkte Bindung steht,
R⁴ für eine direkte Bindung oder ein Wasserstoffatom,
R⁵ und R⁶ gemeinsam für eine gegebenenfalls durch 1-2 Hydroxy- oder 1-3 C₁-C₄-Alkylgruppen substituierte Dimethylen- oder Trimethylen-methingruppe (falls Z für den Rest -CO₂H steht) bzw. gemeinsam für eine gegebenenfalls durch 1-2 Hydroxy- oder 1-3 C₁-C₄-Alkylgruppen substituierte Trimethylen- oder Tetraamethylengruppe (falls Z für die Gruppe 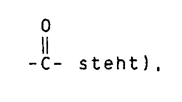 stehen,
mit der Maßgabe, daß nicht gleichzeitig x und w für die Ziffer 0 und V für eine C₀-Alkylenkette stehen und daß Z nur dann für die Gruppe 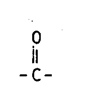 steht, wenn R⁴ gleichzeitig ein Wasserstoffatom bedeutet, und daß Z nur dann für den Rest -CO₂H steht, wenn R⁴ gleichzeitig eine direkte Bindung und s die Ziffer 1 bedeuten,
R³ für den Rest -CH(R¹)CH₃ oder -CH₂CH₂R¹
stehen, mit der Maßgabe, daß R¹ und R² nicht gleichzeitig für die Hydroxygruppe stehen, daß falls R¹ und R² jeweils für die Gruppe -NH(CH₂)ᵣNH₂ mit r in der Bedeutung der Ziffern 2, 3, 4 oder 6 stehen der Substituent R¹ im Rest R³ nicht ebenfalls für diese Gruppe steht, daß der Substituent R¹ im Rest R³ nur für Wasserstoff steht, wenn W in R¹ und R² die Gruppierung V-K bedeutet und daß gewünschtenfalls ein Teil der CO₂H-Gruppen als Ester und/oder Amid sowie die in 3- und/oder 8-Stellung vorhandene Hydroxygruppe gewünschtenfalls als Ether oder Ester vorliegt, sowie gegebenenfalls Kationen anorganischer und/oder organischer Basen, Aminosäuren oder Aminosäureamide.

2. Komplexverbindungen gemäß Anspruch 1, dadurch gekennzeichnet, daß sie mindestens ein Ion eines Elements der Ordnungszahlen 21-29, 42, 44 oder 57-83 enthalten.

3. Komplexverbindungen gemäß Anspruch 1, dadurch gekennzeichnet, daß sie mindestens ein Ion eines Elements der Ordnungszahlen 5, 13, 27, 29, 31, 32, 37-39, 43, 49, 62, 64, 70 oder 77 enthalten.

4. Komplexverbindungen gemäß Anspruch 1, dadurch gekennzeichnet, daß der Porphyrinligand kein Metallion enthält.

5. Komplexverbindungen gemäß Anspruch 1, dadurch gekennzeichnet, daß V für eine
-(CH₂)₂NH-; -CH₂-O-C₆H₄-CH₂-; -CH₂-CH(OH)-CH₂-O-C₆H₄-CH₂-; -C(=NH)-O-C₆H₄-CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂; -CH₂-CO-NH-(CH₂)₃-O-CH₂-; -CH₂-CO-NH-NH-; -CH₂-CO-NH-(CH₂)₂-; -CH₂-CO-NH(CH₂)₁₀-; -CH₂-CO-NH-(CH₂)₂-S-; -(CH₂)₄-NH-CO-(CH₂)₈-; -CH₂-CO-NH-(CH₂)₃-NH; -(CH₂)₃-NH-; -(CH₂)NH-C(=S)-NH-C₆H₄-CH₂-; -CO-CH(NHK)-CH₂NHK-; -CH(CH₃)-S-(CH₂)₂-NH-; -(CH₂)₂NH-CO-CH₂-(OCH₂CH₂)₄₃-OCH₂-gruppe steht.

6. Pharmazeutische Mittel enthaltend mindestens eine Komplexverbindung nach Anspruch 1, gegebenenfalls mit den in der Galenik üblichen Zusätzen.

7. Verwendung von mindestens einem Metallkomplex gemäß Anspruch 2 für die Herstellung von Mitteln für die NMR-, Röntgen- oder Ultraschall-Diagnostik.

8. Verwendung von mindestens einem Metallkomplex gemäß Anspruch 3 für die Herstellung von Mitteln für die Radio-Diagnostik,

9. Verfahren zur Herstellung von Komplexverbindungen gemäß Anspruch 1, dadurch gekennzeichnet, daß man Porphyrine der allgemeinen Formel I' 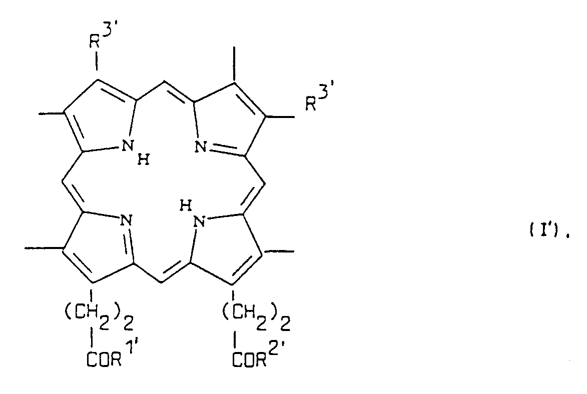 worin
R^{1'} und R^{2'} jeweils für die OH- oder O-C₁-C₆-Alkyl-Gruppe und
R^{3'} für den Rest -CH(U)CH₃ oder -CH₂CH₂-U mit U in der Bedeutung einer OH- oder O-C₁-C₆-Alkyl-Gruppe oder eines Nucleofugs Nf
stehen
in an sich bekannter Weise, gegebenenfalls wiederholt, mit Verbindungen der allgemeinen Formel II
Y-(NH)ₓ-[Q-(NH)_{y}]_{w}-V¹-H (II),
worin
Y für ein Wasserstoffatom, für Nf oder eine Schutzgruppe und
V¹ für V (falls V² für eine C₀-Alkylenkette steht, siehe unten), oder zusammen mit V² (siehe unten) für V steht,
umsetzt und anschließend, gegebenenfalls nach Abspaltung der Schutzgruppen, gewünschtenfalls
a) die pyrrolischen NH's durch das gewünschte Metallatom substituiert, gegebenenfalls noch vorhandene Schutzgruppen entfernt und anschließend gewünschtenfalls den Rest V²-K, worin V² für V oder für eine aktivierte Form von V (falls V¹ für eine C₀-Alkylenkette steht) oder zusammen mit V¹ für V steht, einführt und danach gewünschtenfalls mit mindestens einem Metalloxid oder Metallsalz eines Elements der Ordnungszahlen 21-32, 37-39, 42-44, 49, 50 oder 57-83 umsetzt oder
b) den Substituenten V²-K, worin V² für V oder für eine aktivierte Form von V (falls V¹ für eine C₀-Alkylenkette steht) oder zusammen mit V¹ für V steht, einführt, gewünschtenfalls mit mindestens einem Metalloxid oder Metallsalz eines Elements der Ordnungszahlen 21-32, 37-39, 42-44, 49, 50 oder 57-83 umsetzt und anschließend gewünschtenfalls die pyrrolischen NH's durch ein Metallatom substituiert oder
c) den Substituenten V²-K, worin V² für V oder für eine aktivierte Form von V (falls V¹ für eine C₀-Alkylenkette steht) oder zusammen mit V¹ für V steht, einführt, die pyrrolischen NH's gewünschtenfalls durch ein Metallatom substituiert und anschließend gewünschtenfalls mit mindestens einem Metalloxid oder Metallsalz eines Elements der Ordnungszahlen 21-32, 37-39, 42-44, 49, 50 oder 57-83 umsetzt, wobei zur Herstellung von zwei Porphyrin-Ringe enthaltenden Komplexverbindungen 2 Mole des nach Umsetzung von I' mit II erhaltenen Porphyrins mit einem Komplexbildner K, der zwei V²-Reste enthält, umgesetzt werden, und gegebenenfalls anschließend in den nach a), b) oder c) erhaltenen Komplexverbindungen noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester und/oder Amide und/oder vorhandene Hydroxygruppen in Ether oder Ester überführt.

10. Verfahren zur Herstellung der pharmazeutischen Mittel gemäß Anspruch 6, dadurch gekennzeichnet, daß man die in Wasser oder physiologischer Salzlösung gelöste oder suspendierte Komplexverbindung, gegebenenfalls mit den in der Galenik üblichen Zusätzen, in eine für die enterale oder parenterale Applikation geeignete Form bringt.

## Claims

1. Porphyrin complex compounds, consisting of at least one ion of an element of atomic numbers 5, 13, 21-32, 37-39, 42-44, 49, 50 or 57-83 and at least one porphyrin ligand of the general formula I 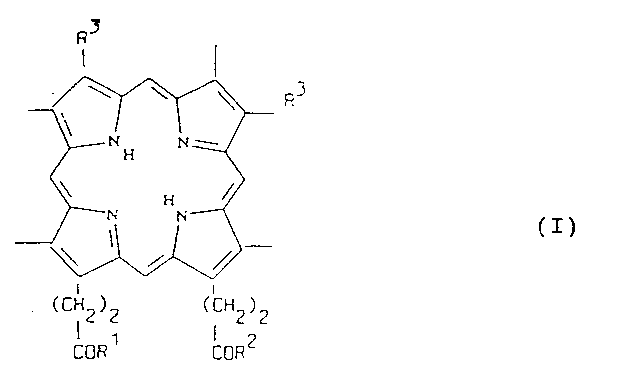 wherein
R¹ and R², independently of one another, represent the group OH or a group (NH)ₓ-[Q(NH)_{y}]_{w}-W,
in which
x and y represent the number 0, 1 or 2,
w represents the number 0 or 1,
Q represents a C₀-C₂₀alkylene group,
W represents a hydrogen atom or the group V-K,
in which
V represents a straight-chained or branched, saturated or unsaturated C₀-C₂₀alkylene group that optionally contains imino, polyethyleneoxy, phenyleneoxy, phenyleneimino, amido, hydrazido, ester group(s), oxygen, sulphur and/or nitrogen atom(s), and that is optionally substituted by hydroxy, mercapto, imino, epoxy, oxo, thioxo and/or NHK group(s),
K represents a hydrogen atom or a complexing agent of the general formula IA, IB, IC or ID 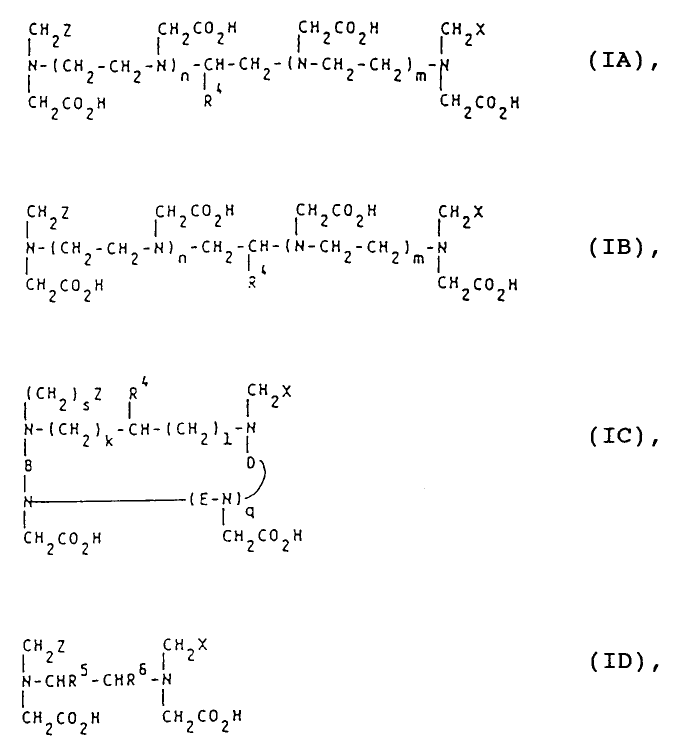 with the proviso that either Z or R⁴ is bonded to V, in which
n and m each represent the number 0, 1, 2, 3 or 4,
where the sum of n and m is not more than 4,
k represents the number 1, 2, 3, 4 or 5,
l represents the number 0, 1, 2, 3, 4 or 5,
q represents the number 0, 1 or 2,
s represents the number 0 or 1,
B, D and E, which are the same or different, each represent a group 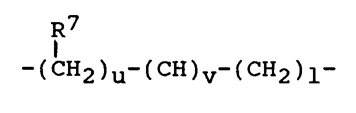 in which
R⁷ represents hydrogen or a straight-chained or branched, saturated or unsaturated C₁-C₂₀alkyl group that optionally contains oxygen and/or nitrogen atom(s) and that is optionally substituted by hydroxy and/or amino group(s),
u represents the number 0, 1, 2, 3, 4 or 5,
v represents the number 0 or 1, B, D and E each containing at least 2 and not more than 5 carbon atoms,
Z represents the radical -CO₂H or the group 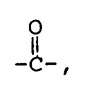
X represents the radical -CO₂H or a radical 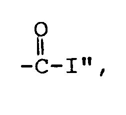 where I'' represents a further porphyrin ligand of the general formula I, in which the radical W in R¹ and R² represents a direct bond,
R⁴ represents a direct bond or a hydrogen atom,
R⁵ and R⁶ together represent a dimethylene- or trimethylene-methine group optionally substituted by 1 or 2 hydroxy groups or by from 1 to 3 C₁-C₄alkyl groups (when Z represents the radical -CO₂H) or together represent a trimethylene or tetramethylene group optionally substituted by 1 or 2 hydroxy groups or by from 1 to 3 C₁-C₄alkyl groups (when Z represents the group 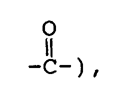
with the proviso that x and w do not represent the number 0 at the same time as V represents a C₀alkylene chain, and that Z represents the group 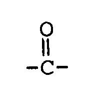 only when R⁴ at the same time represents a hydrogen atom, and that Z represents the radical -CO₂H only when R⁴ at the same time represents a direct bond and s represents the number 1,
R³ represents the radical -CH(R¹)CH₃ or -CH₂CH₂R¹,
with the proviso that R¹ and R² do not simultaneously represent the hydroxy group, that, when R¹ and R² each represent the group -NH(CH₂)ᵣNH₂, where r represents the number 2, 3, 4 or 6, the substituent R¹ in the radical R³ does not likewise represent that group, that the substituent R¹ in the radical R³ represents hydrogen only when W in R¹ and R² represents the group V-K, and that, if desired, some of the CO₂H groups may be in the form of esters and/or amides and the hydroxy group in the 3- and/or 8-position may, if desired, be in the form of an ether or ester,
and, optionally, cations of inorganic and/or organic bases, amino acids or amino acid amides.

2. Complex compounds according to claim 1, characterised in that they contain at least one ion of an element of atomic numbers 21-29, 42, 44 or 57-83.

3. Complex compounds according to claim 1, characterised in that they contain at least one ion of an element of atomic numbers 5, 13, 27, 29, 31, 32, 37-39, 43, 49, 62, 64, 70 or 77.

4. Complex compounds according to claim 1, characterised in that the porphyrin ligand does not contain a metal ion.

5. Complex compounds according to claim 1, characterised in that V represents a group -(CH₂)₂NH-;
-CH₂-O-C₆H₄-CH₂-; -CH₂-CH(OH)-CH₂-O-C₆H₄-CH₂-; -C(=NH)-O-C₆H₄-CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂-; -CH₂-CO-NH-(CH₂)₃-O-CH₂-; -CH₂-CO-NH-NH-; -CH₂-CO-NH-(CH₂)₂-; -CH₂-CO-NH(CH₂)₁₀-; -CH₂-CO-NH-(CH₂)₂-S-; -(CH₂)₄-NH-CO-(CH₂)₈-; -CH₂-CO-NH-(CH₂)₃-NH-; -(CH₂)₃-NH-; -(CH₂)NH-C(=S)-NH-C₆H₄-CH₂-; -CO-CH(NHK)-CH₂NHK-; -CH(CH₃)-S-(CH₂)₂-NH-; -(CH₂)₂NH-CO-CH₂-(OCH₂CH₂)₄₃-OCH₂.

6. Pharmaceutical agents comprising at least one complex compound according to claim 1, optionally together with additives customary in galenic pharmacy.

7. Use of at least one metal complex according to claim 2 for the preparation of agents for NMR, X-ray or ultra-sound diagnostics.

8. Use of at least one metal complex according to claim 3 for the preparation of agents for radiodiagnostics.

9. Process for the preparation of complex compounds according to claim 1, characterised in that porphyrins of the general formula I' 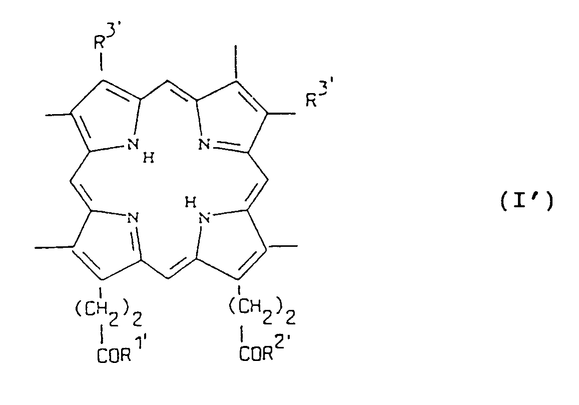 wherein
R^{1'} and R^{2'} each represent the group OH or the group O-C₁-C₆alkyl, and
R^{3'} represents the radical -CH(U)CH₃ or -CH₂CH₂-U, in which U represents the group OH or the group O-C₁-C₆alkyl or a nucleofugal group Nf,
are reacted in a manner known per se, if required repeatedly, with compounds of the general formula II
Y-(NH)ₓ-[Q-(NH)_{y}]_{w}-V¹-H (II)
wherein
Y represents a hydrogen atom, Nf or a protecting group, and
V¹ represents V (when V² represents a C₀alkylene chain, see below) or together with V² (see below) represents V,
and then, optionally after removal of the protecting groups, if desired
a) the pyrrolic NH's are substituted by the desired metal atom, any protecting groups that are still present are removed, and then, if desired, the radical V²-K, in which V² represents V or an activated form of V (when V¹ represents a C₀alkylene chain) or together with V¹ represents V, is introduced, and then, if desired, the whole is reacted with at least one metal oxide or metal salt of an element of atomic numbers 21-32, 37-39, 42-44, 49, 50 or 57-83, or
b) the substituent V²-K, in which V² represents V or an activated form of V (when V¹ represents a C₀alkylene chain) or together with V¹ represents V, is introduced, if desired the whole is reacted with at least one metal oxide or metal salt of an element of atomic numbers 21-32, 37-39, 42-44, 49, 50 or 57-83 and then, if desired, the pyrrolic NH's are substituted by a metal atom, or
c) the substituent V²-K, in which V² represents V or an activated form of V (when V¹ represents a C₀alkylene chain) or together with V¹ represents V, is introduced, the pyrrolic NH's are, if desired, substituted by a metal atom and then, if desired, the whole is reacted with at least one metal oxide or metal salt of an element of atomic numbers 21-32, 37-39, 42-44, 49, 50 or 57-83, 2 moles of the porphyrin obtained after reaction of I' with II being reacted with a complexing agent K containing two radicals V² in order to prepare complex compounds containing two porphyrin rings,
and then, optionally, any acidic hydrogen atoms that are still present in the complex compounds obtained according to a), b) or c) are replaced by cations of inorganic and/or organic bases, amino acids or amino acid amides, or the corresponding acid groups are converted either wholly or partially into esters and/or amides, and/or hydroxy groups that are present are converted into ethers or esters.

10. Process for the preparation of the pharmaceutical agents according to claim 6, characterised in that the complex compound dissolved or suspended in water or physiological saline solution, optionally together with additives customary in galenic pharmacy, is brought into a form suitable for enteral or parenteral administration.

## Revendications

1. Composés complexes de porphyrine, comprenant au moins un ion d'un élément des nombres atomiques 5, 13, 21 - 32, 37 - 39, 42 - 44, 49, 50 ou 57 - 83 et au moins un ligand de porphyrine répondant à la formule générale I 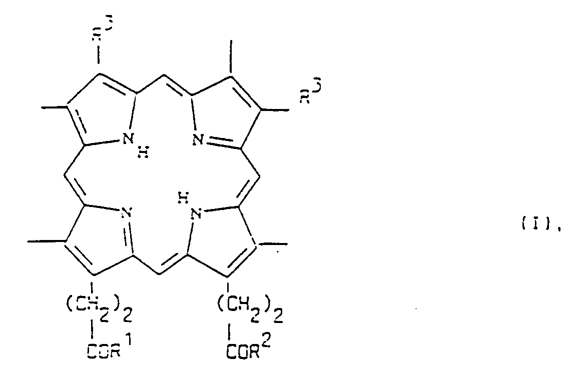 dans laquelle
R¹ et R² représentent indépendamment l'un de l'autre le groupe OH- ou (NH)x-[Q-(NH)_{y}]_{w}-W-
où
X et y représentent les chiffres 0, 1 ou 2,
w représente les chiffres 0 ou 1,
Q représente un groupe alkylène en C₀-C₂₀,
W représente un atome d'hydrogène ou le groupement V-K, où
V représente un groupe alkylène C₀-C₂₀ linéaire, ramifié, saturé ou insaturé, contenant éventuellement un ou des groupes imino, polyéthylèneoxy, phénylèneoxy, phénylèneimino, amido, hydrazido, ester ou un ou des atomes d'oxygène, de soufre et/ou d'azote et substitué éventuellement par un ou des groupes hydroxy, mercapto, imino, époxy, oxo, thioxo et/ou NHK,
K représente un atome d'hydrogène ou un complexant répondant aux formules générales IA, IB, IC ou ID 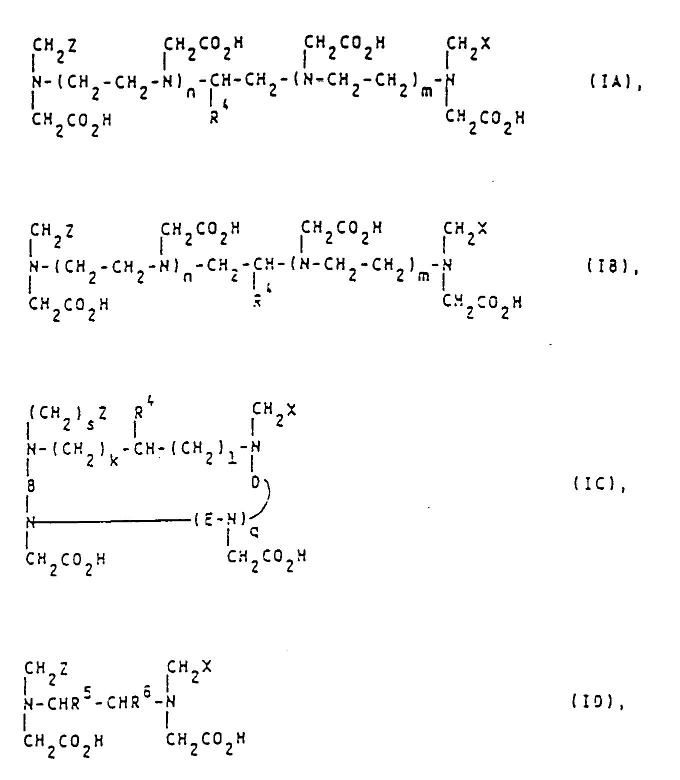 à la condition que soit Z soit R⁴ soit fixé sur V,
n et m représentant chacun les chiffres 0, 1, 2, 3 ou 4, n et m ensemble ne formant pas plus que 4,
k représentant les chiffres 1, 2, 3, 4 ou 5,
l représentant les chiffres 0, 1, 2, 3, 4 ou 5,
q représentant les chiffres 0, 1 ou 2,
s représentant les chiffres 0 ou 1,
B, D et E, qui sont semblables ou différents, représentant chacun le groupe 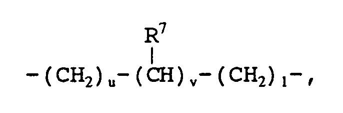 où R⁷ a la signification d'un atome d'hydrogène ou d'un groupe alkyle en C₁-C₂₀ saturé ou insaturé, linéaire ou ramifié, contenant éventuellement un ou des atomes d'oxygène et/ou d'azote et substitué éventuellement par un ou des groupes hydroxy et/ou amino,
u représente les chiffres 0, 1, 2, 3, 4 ou 5,
v représente les chiffres 0 ou 1,
B, D et E contenant chacun au moins 2 et au maximum 5 atomes de carbone,
Z représente le radical -CO₂H ou le groupe 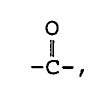 X représente le radical -CO₂H ou le groupe 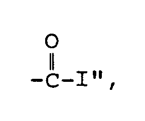 où I'' a la signification d'un autre ligand de porphyrine répondant à la formule générale I, le radical W contenu dans R¹ et R² représentant ici une liaison directe,
R⁴ représente une liaison directe ou un atome d'hydrogène,
R⁵ et R⁶ représentent conjointement un groupe diméthylène-méthyne ou triméthylène-méthyne éventuellement substitué par 1-2 groupes hydroxy ou 1-3 groupes alkyle en C₁-C₄ (dans le cas où Z représente le radical -CO₂H) ou conjointement un groupe triméthylène ou tétraméthylène, éventuellement substitué par 1-2 groupes hydroxy ou 1-3 groupes alkyle en C₁-C₄ (dans le cas où Z représente le groupe 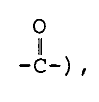 avec la condition qu'il n'y ait pas simultanément x et w représentant le chiffre 0 et V une chaîne alkylène en C₀ et que Z ne représente le groupe 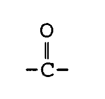 que lorsque R⁴ représente simultanément un atome d'hydrogène, et que Z ne représente le radical -CO₂H que lorsque R⁴ représente simultanément une liaison directe et s le chiffre 1,
R³ représente le radical -CH(R¹)CH₃ ou -CH₂CH₂R¹, à la condition que R¹ et R² ne représentent pas simultanément le groupe hydroxy, que, dans le cas où R¹ et R² représentent chacun le groupe -NH(CH₂)ᵣNH₂ avec r ayant la signification des chiffres 2, 3, 4 ou 6, le substituant R¹ dans le radical R³ ne représente pas également ce groupe, que le substituant R¹ dans le radical R³ ne représente de l'hydrogène que lorsque W dans R¹ et R² représente le groupement V-K et que éventuellement une partie des groupes CO₂H se présente sous la forme d'ester ou/ou d'amide et que le groupe hydroxy présent en position 3 et/ou 8 se présente éventuellement sous la forme d'un éther ou d'un ester, ainsi qu'éventuellement des cations de bases inorganiques et/ou organiques, d'acides aminés ou d'amides d'acides aminés.

2. Composés complexes suivant la revendication 1, caractérisés en ce qu'ils contiennent au moins un ion d'un élément des nombres atomiques 21-29, 42, 44 ou 57-83.

3. Composés complexes suivant la revendication 1, caractérisés en ce qu'ils contiennent au moins un ion d'un élément des nombres atomiques 5, 13, 27, 29, 31, 32, 37-39, 43, 49, 62, 64, 70 ou 77.

4. Composés complexes suivant la revendication 1, caractérisés en ce que le ligand de porphyrine ne contient pas d'ion métallique.

5. Composés complexes suivant la revendication 1, caractérisés en ce que V représente un groupe
-(CH₂)₂NH-, -CH₂-O-C₆H₄-CH₂-, -CH₂-CH(OH)-CH₂-O-C₆H₄-CH₂-, -C(=NH)-O-C₆H₄-CH₂-, -(CH₂)₄-NH-CO-CH₂-O-C₆H₄-CH₂-, -(CH₂)₄-NH-CH₂-CH(OH)-CH₂-O-C₆H₄-CH₂-, -CH₂)₃-O-C₆H₄-CH₂-, -CH₂-CO-NH-(CH₂)₃-O-CH₂-, -CH₂-CO-NH-NH-, -CH₂-CO-NH-(CH₂)₂-, -CH₂-CO-NH(CH₂)₁₀-, -CH₂-CO-NH-(CH₂)-S-, -(CH₂)₄-NH-CO-(CH₂)₈-, -CH₂-CO-NH-(CH₂)₃-NH-, -(CH₂)₃-NH-, -(CH₂)NH-C(=S)-NH-C₆H₄-CH₂-, -CO-CH(NHK)-CH₂NHK-, -CH(CH₃)-S-(CH₂)₂-NH-, -(CH₂)₂NH-CO-CH₂-(OCH₂CH₂)₄₃-OCH₂-.

6. Produits pharmaceutiques contenant au moins un composé complexe suivant la revendication 1, éventuellement avec des additifs courants en pharmacie galénique.

7. Utilisation d'au moins un complexe métallique suivant la revendication 2 pour la préparation de produits pour le diagnostique à la RMN, aux rayons X ou aux ultrasons.

8. Utilisation d'au moins un complexe métallique suivant la revendication 3, pour la préparation de produits pour le radio-diagnostique.

9. Procédé de préparation de composés complexes suivant la revendication 1, caractérisé en ce qu'on fait réagir des porphyrines répondant à la formule générale I' 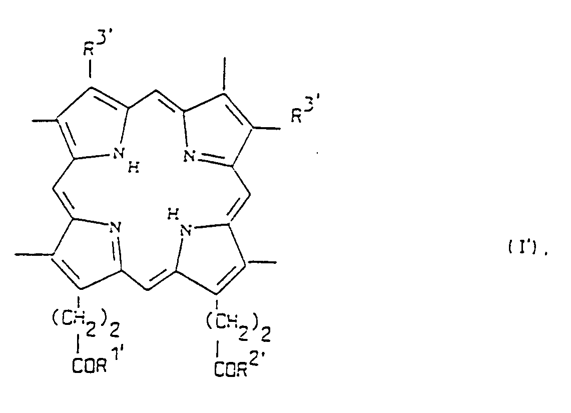 dans laquelle
R^{1'} et R^{2'} représentent chacun le groupe OH- ou O-alkyle en C₁-C₆ et
R^{3'} représente le radical -CH(U)CH₃ ou -CH₂CH₂-U où U a la signification d'un groupe OH ou d'un groupe O-alkyle en C₁-C₆ ou d'un nucléofuge Nf,
d'une manière connue en soi, éventuellement de façon répétée, avec des composés répondant à la formule générale II
Y-(NH)ₓ-[Q-(NH)_{y}]_{w}-V¹-H (II),
dans laquelle
Y représente un atome d'hydrogène, Nf ou un groupe de protection, et
V¹ représente V (dans le cas où V² représente une chaîne alkylène C₀, voir ci-dessous), ou, conjointement à V² (voir ci-dessous), V,
et ensuite, éventuellement après séparation des groupes de protection, éventuellement
a) on substitue les NH pyrroliques par l'atome métallique souhaité, on élimine éventuellement des groupes de protection encore présents et ensuite éventuellement on introduit le radical V²-K où V² représente V ou une forme activée de V (dans le cas où V¹ représente une chaîne alkylène C₀) ou, conjointement à V¹, V, et ensuite éventuellement on fait réagir avec au moins un oxyde métallique ou sel métallique d'un élément des nombres atomiques 21-32, 37-39, 42-44, 49, 50 ou 57-83, ou
b) on introduit le substituant V²-K, où V² représente V ou une forme activée de V (dans le cas où V¹ représente une chaîne alkylène C₀) ou, conjointement à V¹, V, on fait éventuellement réagir avec au moins un oxyde métallique ou sel métallique d'un élément des nombres atomiques 21-32, 37-39, 42-44, 49, 50 ou 57-83 et ensuite éventuellement on substitue les NH pyrroliques par un atome métallique, ou
c) on introduit le substituant V²-K, où V² représente V ou une forme activée de V (dans le cas où V¹ représente une chaîne alkylène C₀) ou, conjointement à V¹ V, on substitue les NH pyrroliques éventuellement par un atome métallique et ensuite éventuellement on fait réagir avec au moins un oxyde métallique ou sel métallique d'un élément des nombres atomiques 21-32, 37-39, 42-44, 49, 50 ou 57-83, 2 moles de la porphyrine obtenue après réaction de I' avec II étant, pour la préparation de composés complexes contenant deux noyaux de porphyrine, amenées à réagir avec un complexant K qui contient deux radicaux V², et éventuellement ensuite on substitue, dans les composés complexes obtenus selon a), b) ou c), des atomes d'hydrogène acides encore présents par des cations de bases inorganiques ou organiques, d'acides aminés, ou d'amides d'acides aminés et respectivement on convertit les groupes acides correspondants, totalement ou partiellement, en esters et/ou amides et/ou des groupes hydroxy présents en éthers ou esters.

10. Procédé de préparation de produits pharmaceutiques suivant la revendication 6, caractérisé en ce qu'on amène sous une forme appropriée pour l'application entérale ou parentérale le composé complexe en solution ou suspension dans de l'eau ou du sérum physiologique, éventuellement avec les additifs courants en pharmacie galénique.
